(19) Europäisches Patentamt
European Patent Office
Office européen des brevets

(11) EP 0 919 542 B1

(12) # EUROPEAN PATENT SPECIFICATION

(45) Date of publication and mention of the grant of the patent:
**23.08.2006 Bulletin 2006/34**

(21) Application number: **98122107.0**

(22) Date of filing: **23.11.1998**

(51) Int Cl.:
*C07C 233/64* (2006.01) *C07C 235/42* (2006.01)
*C07C 317/32* (2006.01) *C07C 323/42* (2006.01)
*C07D 295/192* (2006.01) *A01N 37/18* (2006.01)
*A01N 43/00* (2006.01) *A01N 43/84* (2006.01)
*A01N 57/00* (2006.01)

(54) **Phthalic acid diamide derivatives, agricultural and horticultural insecticides, and a method for application of the insecticides**

PHTHALSAEUREDIAMIDDERIVATE, INSEKTIZIDE FÜR LANDBAU UND GARTENBAU, UND DEREN VERWENDUNG

DERIVES DIAMIDE DE L'ACIDE PHTHALIQUE, INSECTICIDES POUR L'AGRICULTURE ET HORTICULTURE, ET LEUR UTILISATION

(84) Designated Contracting States:
**CH DE DK ES FI FR GB IT LI**

(30) Priority: **25.11.1997 JP 33939397**

(43) Date of publication of application:
**02.06.1999 Bulletin 1999/22**

(60) Divisional application:
**04009422.9 / 1 447 396**
**06010551.7**

(73) Proprietor: **NIHON NOHYAKU CO., LTD.**
**Chuo-ku**
**Tokyo100-0027 (JP)**

(72) Inventors:
- **Tohnishi, Masanori**
  **Sakai-shi (JP)**
- **Nakao, Hayami**
  **Kawachinagano-shi (JP)**
- **Kohno, Eiji**
  **Habikino-shi (JP)**
- **Nishida, Tateki**
  **Tondabayashi-shi (JP)**
- **Furuya, Takashi**
  **Izumisano-shi (JP)**
- **Shimizu, Toshiaki**
  **Kawachinagano-shi (JP)**
- **Seo, Akira**
  **Hashimoto-shi (JP)**
- **Sakata, Kazuyuki**
  **Kawachinagano-shi (JP)**
- **Fujioka, Shinsuke**
  **Kawachinagano-shi (JP)**
- **Kanno, Hideo**
  **Ibaraki-shi (JP)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 0 119 428**
**DE-A- 3 802 175**
**US-A- 3 502 685**
**US-A- 4 694 016**
**US-A- 4 732 845**

- **PATENT ABSTRACTS OF JAPAN vol. 011, no. 003 (C-395), 7 January 1987 (1987-01-07) & JP 61 180753 A (NIPPON KAYAKU CO LTD), 13 August 1986 (1986-08-13)**
- **PATENT ABSTRACTS OF JAPAN vol. 015, no. 466 (P-1280), 26 November 1991 (1991-11-26) & JP 03 198049 A (FUJI PHOTO FILM CO LTD), 29 August 1991 (1991-08-29)**
- **CHEMICAL ABSTRACTS, vol. 124, no. 6, 5 February 1996 (1996-02-05) Columbus, Ohio, US; abstract no. 56894r, PERRY ROBERT J. ET AL.: "Polyimide formation through the palladium-mediated carbonylation..." page 12; column 1; XP002095523 & MACROMOLECULES, vol. 29, no. 3, 1996, pages 1014-1020,**
- **CHEMICAL ABSTRACTS, vol. 123, no. 26, 25 December 1995 (1995-12-25) Columbus, Ohio, US; abstract no. 343359e, HALL, NIGEL: page 180; column 2; XP002095524 & WO 95 20014 A (ZENECA LTD)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 919 542 B1

- CHEMICAL ABSTRACTS, vol. 120, no. 21, 23 May 1994 (1994-05-23) Columbus, Ohio, US; abstract no. 269853f, BEELY, NIGEL ET AL.: page 1012; column 1; XP002095525 & WO 93 25517 A (CELLTECH LTD)
- CHEMICAL ABSTRACTS, vol. 113, no. 19, 5 November 1990 (1990-11-05) Columbus, Ohio, US; abstract no. 171822a, ISMAIL, M. FEKRY ET AL.: "Reaction of N-arylphthalisoimidium perchlorates with amines ..." page 686; column 2; XP002095526 & ZEITSCHRIFT FUR NATURFORSCHUNG, TEIL B: CHEM. SCI., vol. 45, no. 5, 1990, pages 707-710,
- CHEMICAL ABSTRACTS, vol. 109, no. 5, 1 August 1988 (1988-08-01) Columbus, Ohio, US; abstract no. 37697c, GANIN, E. V. ET AL.: "N-substituted isophthalimide formation." page 604; column 1; XP002095527 & UKR. KHIM. ZH., vol. 53, no. 9, 1987, pages 964-967,
- CHEMICAL ABSTRACTS, vol. 91, no. 9, 27 August 1979 (1979-08-27) Columbus, Ohio, US; abstract no. 74313e, DE SILVA, S. OSMUND ET AL.: "Directed lithiation ...." page 569; column 1; XP002095528 & TETRAHEDRON LETTERS., vol. 51, 1978, pages 5099-5102, OXFORD GB
- CHEMICAL ABSTRACTS, vol. 68, no. 3, 15 January 1968 (1968-01-15) Columbus, Ohio, US; abstract no. 12654h, EDITH G. DIAZ DE TORANZO ET AL.: "Syntheses of unsymmetric o-phthalic diamides." page 1198; column 2; XP002095529 & JOURNAL OF MEDICINAL CHEMISTRY., vol. 10, no. 5, 1967, pages 982-983, WASHINGTON US
- CHEMICAL ABSTRACTS, vol. 70, no. 15, 14 April 1969 (1969-04-14) Columbus, Ohio, US; abstract no. 68102k, RESPLANDY, ALBERT ET AL.: "Synthesis of 6-phenanthridone-7-carboxylic acids from N-arylphthalamic acids." page 353; column 1; XP002095530 & BULLETIN DE LA SOCIETE CHIMIQUE DE FRANCE., vol. 12, 1968, pages 4947-4953, PARIS FR
- CHEMICAL ABSTRACTS, vol. 126, no. 21, 26 May 1997 (1997-05-26) Columbus, Ohio, US; abstract no. 277494d, page 673; column 1; XP002128153 & JP 09 059236 A (DAIICHI SEIYAKU )
- CHEMICAL ABSTRACTS, vol. 124, no. 3, 15 January 1996 (1996-01-15) Columbus, Ohio, US; abstract no. 29077v, ISMAIL, M. FEKRY ET AL.: "Studies on the mode of ring-opening of N-aryl phthalisoimidium perchlorates." page 913; column 2; XP002128154 & POL. J. CHEM., vol. 69, no. 8, 1995, pages 1114-1119,
- CHEMICAL ABSTRACTS, vol. 122, no. 19, 8 May 1995 (1995-05-08) Columbus, Ohio, US; abstract no. 239480f, RANADIVE, V. B. ET AL.: "Nucleophilic reactions of N-hydroxy-,methoxy-, 2,3-epoxypropoxy-phthalimides" page 1039; column 1; XP002128155 & INDIAN J. CHEM., SECT. B: ORG. CHEM. INCL. MED. CHEM., vol. 33B, no. 12, 1994, pages 1175-1177,
- CHEMICAL ABSTRACTS, vol. 108, no. 1, 4 January 1988 (1988-01-04) Columbus, Ohio, US; abstract no. 5636v, CREMLYN, R. J. ET AL.: "Phthalimidobenzenesulfonyl derivatives" page 533; column 1; XP002128156 & QUIM. NOVA, vol. 8, no. 1, 1985, pages 61-62,
- PATENT ABSTRACTS OF JAPAN vol. 009, no. 119 (C-282), 23 May 1985 (1985-05-23) & JP 60 008247 A (SHOWA DENKO KK), 17 January 1985 (1985-01-17)
- CHEMICAL ABSTRACTS, vol. 79, no. 23, 10 December 1973 (1973-12-10) Columbus, Ohio, US; abstract no. 136746m, IMAI, YOSHIO ET AL.: "Reaction of N-(phenylsulfonyl)phthalimde ...." page 347; column 2; XP002128157 & NIPPON KAGAKU KAISI, vol. 9, 1973, pages 1729-1733,
- CHEMICAL ABSTRACTS, vol. 57, no. 7, 1 October 1962 (1962-10-01) Columbus, Ohio, US; abstract no. 8530g, WALTER RIED ET AL.: "Monoaddition of acetylene to N-substituted phthalimides" page 1962; column 2; XP002128158 & BER., vol. 95, 1962, pages 1562-1564,
- FROEHLICH E.: 'Ueber Derivate des Pseudocumidins' CHEM. BER. vol. 17, 1884, pages 1801 - 1809
- GANIN E.V. ET AL: 'Features of the reaction of phthaloyl chloride with primary amines' J. ORG. CHEM. USSR (ENGL. TRANSL.) vol. 21, no. 9, 1985, pages 1772 - 1777
- KARAT L.D.; STREL'TSOV V.I.: 'Kinetics of the aminolysis of the cyclic imides of 1,2-dicarboxylic acids by piperidine' RUSS. J. ORG. CHEM. vol. 29, no. 2, 1993, pages 293 - 297

## Description

The present invention relates to phthalic acid diamide derivatives, agricultural and horticultural insecticides containing said derivative as an active ingredient, and a method for application of the insecticides.

Japanese Patent Application Nos. 59-163353 and 61-180753 and J.C.S. Perkin I, 1338-1350, (1978), etc. disclose similar phthalic acid diamide derivatives but neither describe nor suggest their usefulness as agricultural and horticultural insecticides.

The present inventors earnestly studied in order to develop a novel agricultural and horticultural insecticide, and consequently found that the phthalic acid diamide derivatives represented by the general formula (I-2) and by the general formule (I-3) of the present invention are novel compounds not known as agricultural and horticultural insecticides in any literature and that said derivatives including the compounds disclosed in the above references can be used for a new purpose as agricultural and horticultural insecticides. Thus, the present invention has been accomplished.

The present invention relates to phthalic acid diamide derivatives of the general formula (1-2)

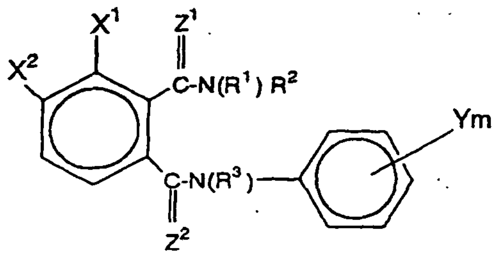

(I-2)

{wherein

$R^1$, $R^2$ and $R^3$ may be the same or different, and are each a hydrogen atom; a $C_{3-}C_6$ cycloalkyl group; a halo-$C_3$-$C_6$ cycloalkyl group or a group of the formula -$A^1$-$Q_1$ (wherein $A^1$ is a $C_1$-$C_8$ alkylene group; a $C_3$-$C_6$ alkenylene group or a $C_3$-$C_6$ alkynylene group;

Q is a hydrogen atom; a halogen atom; a cyano group; a nitro group; a halo-$C_1$-$C_6$ alkyl group; a $C_3$-$C_6$ cycloalkyl group; a halo-$C_3$-$C_6$ cycloalkyl group; a $C_1$-$C_6$ alkoxycarbonyl group; a di-$C_1$-$C_6$ alkoxyphosphoryl group which may be the same or different; a di-$C_1$-$C_6$ alkoxythiophosphoryl group which may be the same or different; a diphenylphosphino group; a diphenylphosphono group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group; a heterocyclic group (wherein the heterocyclic group is defined as below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo- $C_1$-$C_6$ alkyl-sulfonyl group; or a group of the formula -$Z^3$-$R^5$

(wherein $Z^3$ is -O-; -S-; -SO-; -$SO_2$- or a group of the formula -N($R^6$)- (wherein $R^6$ is a hydrogen atom; a $C_1$-$C_6$ alkylcarbonyl group; a halo-$C_1$-$C_6$ alkylcarbonyl group; a $C_1$-$C_6$ alkoxycarbonyl group; a phenylcarbonyl group; a substituted phenylcarbonyl group having at least one substituent which may be the same or different and is selected from the group consisting of a halogen atom, a $C_1$-$C_6$ alkyl group, a halo-$C_{1-}C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenyl $C_1$-$C_4$ alkoxycarbonyl group or a substituted phenyl $C_1$-$C_4$ alkoxycarbonyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group)); and

$R^5$ is a hydrogen atom; a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$ alkynyl group; a halo-$C_3$-$C_6$ alkynyl group; a $C_3$-$C_6$ cycloalkyl group; a halo-$C_3$-$C_6$ cycloalkyl group; a $C_1$-$C_6$ alkylcarbonyl group; a halo- $C_1$-$C_6$ alkylcarbonyl group; a $C_1$-$C_6$ alkoxycarbonyl group; a phenyl group; a substituted phenyl group having at least one substituent which may be the same or different and is selected from

the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenyl $C_1$-$C_4$ alkyl group; a substituted phenyl $C_1$-$C_4$ alkyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); or a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group; a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group); and l is an integer of 1 to 4); further,

$R^1$ and $R^2$ may form a 4 to 7 membered ring by combining to each other, in which the ring may contain the same or different 1 to 3 heteroatoms selected from the group consisting of oxygen atom, sulfur atom and nitrogen atom;

$X^1$ and $X^2$ may be the same or different and are each a halogen atom; a cyano group; a $C_1$-$C_6$ alkyl group; a halo-$C_1$-$C_6$ alkyl group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group and a halo-$C_1$-$C_6$ alkyl-sulfonyl group; further,

$X^1$ and $X^2$ may form a condensed ring with the phenyl ring of phthalic acid diamide derivative (which means naphthalene, tetrahydronaphthalene, indene, indane, quinoline, quinazoline, chroman, isochroman, indole, indoline, benzodioxane, benzodioxole, benzofuran, dihydrobenzofuran, benzothiophene, dihydrobenzothiophene, benzoxazole, benzothiazole, benzimidazole, or indazole) by combining to each other, and said condensed ring may have at least one substituent, which may be the same or different and is selected from the group consisting of a halogen atom; a $C_1$-$C_6$ alkyl group; a halo-$C_1$-$C_6$ alkylgroup; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkylsulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); and a substituted heterocyclic group (wherein the heterocyclic group is the same as defined above) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group;

Y is the same or different, and are each a hydrogen atom; a halogen atom; a cyano group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of —$A^2$-$R^7$ (wherein $A^2$ is -O-; -S-; -SO-; - $SO_2$-; -C(=O)-; -C(=$NOR^8$)- (wherein $R^8$ is a hydrogen atom; a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$ alkynyl group; a $C_3$-$C_6$ cycloalkyl group; a phenyl-$C_1$-$C_4$ alkyl group; or a substituted phenyl-$C_1$-$C_4$ alkyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group)); a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group or a halo-$C_3$-$C_6$ alkynylene group;

(1) when $A^2$ is -O-; -S-; -SO- or -$SO_2$-, then $R^7$ is a halo-$C_3$-$C_6$ cycloalkyl group; a halo-$C_3$-$C_6$ cycloalkenyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl

group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo- $C_1$-$C_6$ alkylsulfonyl group; a heterocyclic group (which is the same as defined below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of the formula $A^3$-$R^9$

(wherein $A^3$ is a $C_1$-$C_6$ alkylene group; a halo-$C_1$-$C_6$ alkylene group; a $C_3$-$C_6$ alkenylene group; a halo-$C_3$-$C_6$ alkenylene group; a $C_3$-$C_9$ alkynylene group or a halo-$C_3$-$C_6$ alkynylene group;

$R^9$ is a hydrogen atom; a halogen atom; a $C_3$-$C_6$ cycloalkyl group; a halo-$C_{3-}C_6$ cycloalkyl group; a $C_1$-$C_6$ alkoxycarbonyl group; a phenyl group; a substituted phenyl group having at least one substituent which may be the same or different and is selected from the group consisting of a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkyl-sulfonyl group; a halo-$C_1$-$C_6$ alkylsulfonyl group or a group of the formula -$A^4$-$R^{10}$

(wherein $A^4$ is -O-; -S-; -SO-; -$SO_2$- or -C(=O)-, and

$R^{10}$ is a $C_1$-$C_6$ alkyl group; a halo- $C_1$-$C_6$ alkyl group; a $C_3$-$C_6$ alkenyl group; a halo-$C_3$-$C_6$ alkenyl group; a $C_3$-$C_6$ cycloalkyl group; a halo-$C_3$-$C_6$ cycloalkyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); or a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group));

(2) when $A^2$ is -C(=O)- or a group of the formula -C(=$NOR^8$)- (wherein $R^8$ is the same as defined the above), then $R^7$ is a $C_1$-$C_8$ alkyl group; a halo $C_1$-$C_6$ alkyl group; a $C_2$-$C_6$ alkenyl group; a halo-$C_2$-$C_6$ alkenyl group; a $C_3$-$C_6$ cycloalkyl group; a halo-$C_3$-$C_6$ cycloalkyl group; a $C_1$-$C_6$ alkoxy group; a $C_1$-$C_6$ alkylthio group; a mono-$C_1$-$C_6$ alkylamino group; a di-$C_1$-$C_6$ alkylamino group which may be the same or different; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenylamino group; a substituted phenylamino group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group, and a halo-$C_1$-$C_6$ alkylsulfonyl group; a heterocyclic group (which is the same as defined the below); or a substituted heterocyclic group (wherein the heterocyclic group is the same as defined the below) having at least one substituent which may be the same or different and is selected from the group consisting of a halogen atom, a $C_1$-$C_6$ alkyl group, a halo- $C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo- $C_1$-$C_6$ alkylsulfonyl group;

(3) when $A^2$ is a $C_1$-$C_6$ alkylene group; a halo-$C_1$-$C_6$ alkylene group; $C_2$-$C_6$ alkenylene group; a halo-$C_2$-$C_6$ alkenylene group; a $C_2$-$C_6$ alkynylene group or a halo-$C_3$-$C_6$ alkynylene group; then $R^7$ is a hydrogen atom; a halogen atom; a $C_3$-$C_6$ cycloalkyl group; a halo-$C_3$-$C_6$ cycloalkyl group; a $C_1$-$C_6$ alkoxycarbonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkyl-sulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$

alkylsulfonyl group; or a group of the formula $-A^5-R^{12}$
(wherein $A^5$ is -O-; -S-; -SO- or $-SO_2-$; and
$R^{12}$ is a $C_3$-$C_6$ cycloalkyl group; a halo-$C_3$-$C_6$ cycloalkyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of the formula $-A^6-R^{14}$
(wherein $A^6$ is a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group; or a halo-$C_3$-$C_6$ alkynylene group; and
$R^{14}$ is a hydrogen atom; a halogen atom; a $C_3$-$C_6$ cycloalkyl group; a halo-$C_3$-$C_6$ cycloalkyl group; a $C_1$-$C_6$ alkoxy group; a halo-$C_1$-$C_6$ alkoxy group; a $C_1$-$C_6$ alkylthio group; a halo- $C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkyl-sulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenoxy group; a substituted phenoxy group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenylthio group; a substituted phenylthio group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); or a substituted heterocyclic group (wherein the heterocyclic ring is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group))); and

m is an integer of 1 to 5;
further, Y may form a condensed ring (which is the same as defined above) by combining together with the adjacent carbon atoms in the phenyl ring, said condensed ring may have at least one substituent, which is the same or different and is selected from the group consisting of a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkylsulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); and a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group;
$Z^1$ and $Z^2$ are each an oxygen atom or a sulfur atom; and

said heterocyclic group is selected from the group consisting of a pyridyl group, pyridine-N-oxide group, pyrimidinyl group, furyl group, tetrahydrofuryl group, thienyl group, tetrahydrothienyl group, tetrahydropyranyl group, tetrahydrothiopyranyl group, oxazolyl group, isoxazolyl group, oxadiazolyl group, thiazolyl group, isothiazolyl group, thiadiazolyl group, imidazolyl group, triazolyl group and pyrazolyl group}.

Preferred compounds are those represented by the general formula (I-3),

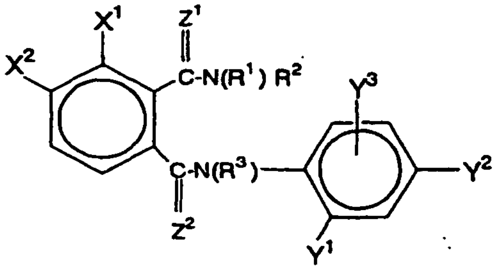

(I-3)

{wherein $R^1$, $R^2$ and $R^3$, I, $X^1$ and $X^2$ are defined as in formula (I-2);

$Y^1$ and $Y^3$ may be the same or different, and are each a hydrogen atom; a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a phenoxy group; a substituted phenoxy group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkyl-sulfonyl group and a halo-$C_1$-$C_6$ alkyl-sulfonyl group; a pyridyloxy group; or a substituted pyridyloxy group having at least one substituent which may be the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group;

$Y^2$ is a hydrogen atom; a halogen atom or a group of the formula -$A^2$-$R^7$

(wherein $A^2$ -O-; -S-; -SO-; -$SO_2$-; a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group; or a halo-$C_3$-$C_6$ alkynylene group; and

(1) when $A^2$ is -O-; -S-; -SO- or -$SO_2$-, then $R^7$ is a halo-$C_3$-$C_6$ cycloalkyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a $C_1$-$C_6$ alkylsulfinyl group and a halo-$C_1$-$C_6$ alkylsulfonyl group; a substituted pyridyloxy group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^3$-$R^9$

(wherein $A^3$ is a halo-$C_1$-$C_6$ alkylene group or a halo-$C_3$-$C_6$ alkenylene group and;

$R^9$ is a hydrogen atom; a halogen atom; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^4$-$R^{10}$

(wherein $A^4$ is -O-; -S-; -SO- or -$SO_2$-

$R^{10}$ is a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$-cycloalkyl group; a halo-$C_3$-$C_6$ cycloalkyl group; a phenyl group; or a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkyl-sulfinyl group and a halo-$C_1$-$C_6$ alkylsulfonyl group));

(2) when $A^2$ is a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group; a halo-$C_3$-$C_6$ alkynylene group; then $R^7$ is a hydrogen atom; a halogen atom; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^5$-$R^{12}$

(wherein $A^5$ is -O-; -S-; -SO- or -$SO_2$-; and

$R^{12}$ is a $C_3$-$C_6$ cycloalkyl group; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^6$-$R^{14}$

(wherein $A^6$ is a $C_1$-$C_6$ alkylene group; a halo-$C_1$-$C_6$ alkylene group; a $C_2$-$C_6$ alkenylene group; a halo-$C_2$-$C_6$ alkenylene group; and

$R^{14}$ is a hydrogen atom; a halogen atom; a halo-$C_3$-$C_6$ cycloalkyl group; a halo-$C_1$-$C_6$ alkoxy group; a halo-$C_1$-$C_6$ alkylthio group; a halo-$C_1$-$C_6$ alkylsulfinyl group; a halo-$C_1$-$C_6$ alkylsulfonyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; a phenoxy group; a substituted phenoxy group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; a phenylthio group; or a substituted phenylthio group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group)); and further,

$Z^1$ and $Z^2$ are defined as in formula (I-2);

agricultural and horticultural insecticides containing as an active ingredient any of the phthalic acid diamide derivatives of the general formula (I-2) or general formula (I-3) including known compounds; and a method for application of the insecticides.

In the definition of the general formula (I-2) and general formula (I-3) representing the phthalic acid diamide derivative of the present invention, the halogen atom includes chlorine atom, bromine atom, iodine atom and fluorine atom. The term "$C_1$-$C_6$ alkyl" means a linear or branched alkyl group of 1 to 6 carbon atoms, such as methyl, ethyl, n-propyl, i-propyl, n-butyl, i-butyl, s-butyl, t-butyl, n-pentyl or n-hexyl. The term "$C_1$-$C_8$ alkylene" means a linear or branched alkylene group of 1 to 8 carbon atoms, such as methylene, ethylene, propylene, trimethylene, dimethylmethylene, tetramethylene, i-butylene, dimethylethylene, pentamethylene, hexamethylene, heptamethylene or octamethylene. The term "halo-$C_1$-$C_6$ alkyl" means a substituted and linear or branched alkyl group of 1 to 6 carbon atoms having as the substituent(s) one or more halogen atoms which may be the same or different.

As the ring which $R^1$ and $R^2$ form by combining to each other, i.e., the 4- to 7-membered ring by combining to each other, in which the ring may contain the same or different 1 to 3 hetero atoms selected from the group consisting of oxygen atom, sulfur atom and nitrogen atom, there can be exemplified azetidine ring, pyrrolidine ring, pyrroline ring, piperidine ring, imidazolidine ring, imidazoline ring, oxazolidine ring, thiazolidine ring, isoxazolidine ring, isothiazolidine ring, tetrahydropyridine ring, piperazine ring, morpholine ring, thiomorpholine ring, dioxazine ring, dithiazine ring, etc.

The phthalic acid diamide derivative of the general formula (I-2) of or the general formula (I-3) of the present invention contains an asymmetric carbon atom or some asymmetric center in the structural formula in some cases or has two optical isomers in some cases. The present invention includes these optical isomers and all mixtures containing the optical isomers in arbitrary proportions.

Preferable examples of each substituent of the phthalic acid diamide derivative of the general formula (I-2) or of the general formula (I-3) of the present invention are as follows. Preferable examples of each of $R^1$ and $R^2$ which may be the same or different are hydrogen atom, $C_1$-$C_6$ alkyl groups such as methyl, ethyl, i-propyl, etc. Preferable examples of $R^3$ are hydrogen atom, and $C_1$-$C_6$ alkyl groups such as methyl, ethyl, n-propyl, i-propyl, n-butyl, etc. Preferable examples of X are halogen atoms, nitro group, halo-$C_1$-$C_6$ alkyl groups, halo-$C_1$-$C_6$ alkoxy groups, halo-$C_1$-$C_6$ alkylthio groups, etc. Preferable examples of Y are halo-$C_1$-$C_6$ alkyl groups, halo-$C_1$-$C_6$ alkoxy groups, halo-$C_1$-$C_6$ alkylthio groups, etc.

The phthalic acid diamide derivative of the general formula (I-2) or general formula (I-3) of the present invention can be produced, for example, by any of the processes illustrated below with respect to general formula (I) and the variations thereof, namely formula (I-1'), formula (I-2') and formula (I-3').

Production process 1.

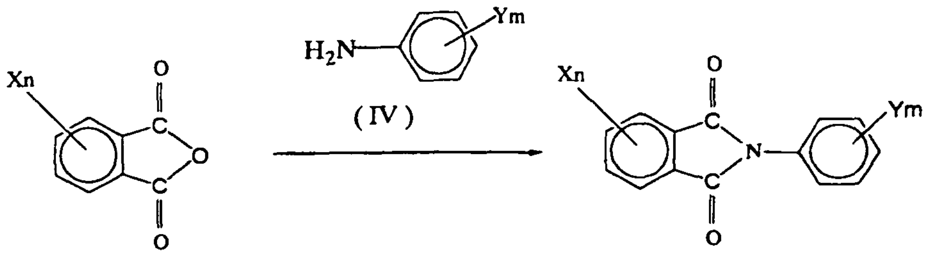


(V) (III)

(III)

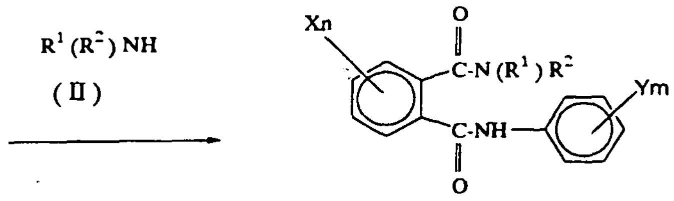


(I-1')

wherein $R^1$, $R^2$, X, Z, Y and m are as defined above and n is 2 with X being attached to the 3 and 4 position.

A phthalic anhydride derivative of the general formula (V) is reacted with an aniline of the general formula (IV) in the presence of an inert solvent to obtain a phthalimide derivative of the general formula (III). The phthalimide derivative (III) is reacted with an amine of the general formula (II) after or without being isolated, whereby a phthalic acid diamide derivative of the general formula (I-1') can be produced.

(1) General formula (V) → general formula (III)

As the inert solvent used in this reaction, any solvent may be used so long as it does not markedly inhibit the progress of the reaction. There can be exemplified aromatic hydrocarbons such as benzene, toluene, xylene, etc.; halogenated hydrocarbons such as dichloromethane, chloroform, carbon tetrachloride, etc., chlorinated aromatic hydrocarbons such as chlorobenzene, dichlorobenzene, etc.; a cyclic or cyclic ethers such as diethyl ether, dioxane, tetrahydrofuran, etc., esters such as ethyl acetate, etc.; amides such as dimethylformamide, dimethylacetamide, etc.; acids such as acetic acid, etc.; dimethyl sulfoxide; and 1,3-dimethyl-2-imidazolidinone. These inert solvents may be used alone or as a mixture thereof.

Since the reaction is an equimolar reaction, it is sufficient that the reactants are used in equimolar amounts, though either of them may be used in excess. If necessary, the reaction may be carried out under dehydrating conditions.

As to the reaction temperature, the reaction can be carried out in a temperature range of room temperature to the reflux temperature of the inert solvent used. Although the reaction time is varied depending on the scale of reaction, the reaction temperature, etc., it may be properly chosen in a range of several minutes to 48 hours.

After completion of the reaction, the desired compound is isolated from the reaction solution containing the desired compound by a conventional method, and if necessary, purified by recrystallization, column chromatography, etc., whereby the desired compound can be produced. The desired compound can be subjected to the subsequent reaction without isolation from the reaction solution.

The phthalic anhydride derivative of the general formula (V) can be produced by the process described in J. Org. Chem., 52, 129 (1987), J. Am. Chem. Soc., 51, 1865 (1929), J. Am. Chem. Soc., 63, 1542 (1941), etc. The aniline of the general formula (IV) can be produced by the process described in J. Org. Chem., 29, 1 (1964), Angew. Chem. Int. Ed. Engl., 24, 871 (1985), Synthesis, 1984, 667, Bulletin of the Chemical Society of Japan, 1973, 2351, DE-2606982, JP-A-1-90163, etc.

(2) General formula (III) → general formula (I-1')

In this reaction, there can be used the inert solvents exemplified above as the inert solvent used in the reaction (1).
Since the reaction is an equimolar reaction, it is sufficient that the reactants are used in equimolar amounts, though the amine of the general formula (II) may be used in excess.
As to the reaction temperature, the reaction can be carried out in a temperature range of room temperature to the reflux temperature of the inert solvent used. Although the reaction time is varied depending on the scale of reaction, the reaction temperature, etc., it may be properly chosen in a range of several minutes to 48 hours.
After completion of the reaction, the desired compound is isolated from the reaction solution containing the desired compound by a conventional method, and if necessary, purified by recrystallization, column chromatography, etc., whereby the desired compound can be produced.

Production process 2.

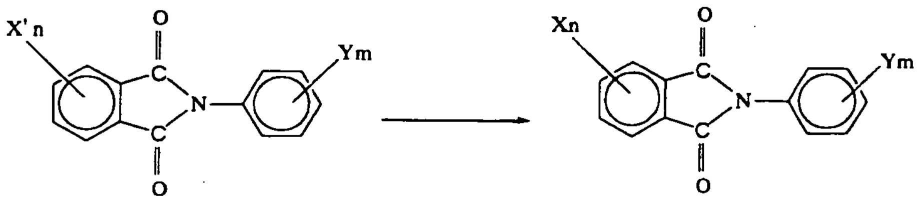

(III-1) (III)

(III)

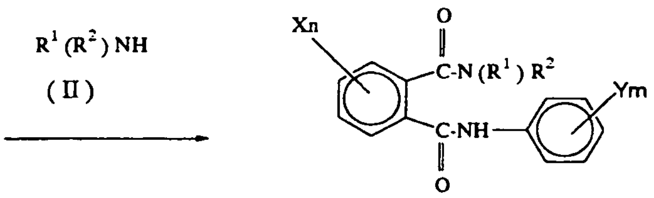


(I-1')

wherein $R^1$, $R^2$, X, Y and m are as defined above, n is 2 with X being attached to the 3 and 4-position, and X' is a halogen atom or a nitro group, provided that X is other than a hydrogen atom or a nitro group.
A phthalimide derivative of the general formula (III-1) is reacted with a reactant corresponding to X in the presence of an inert solvent to obtain a phthalimide derivative of the general formula (III). The phthalimide derivative (III) is reacted with an amine of the general formula (II) after or without being isolated, whereby a phthalic acid diamide derivative of the general formula (I-1') can be produced.

(1) General formula (III-1) → general formula (III)

This reaction can be carried out according to the methods described in J. Org. Chem., 42, 3415 (1977), Tetrahedron, 25, 5921 (1969), Synthesis, 1984, 667, Chem. Lett., 1973, 471, J. Org. Chem., 39, 3318 (1974), J. Org. Chem., 39, 3327 (1974), etc.

(2) General formula (III) → general formula (I-1')

This reaction can be carried out according to production process 1-(2).

Production process 3

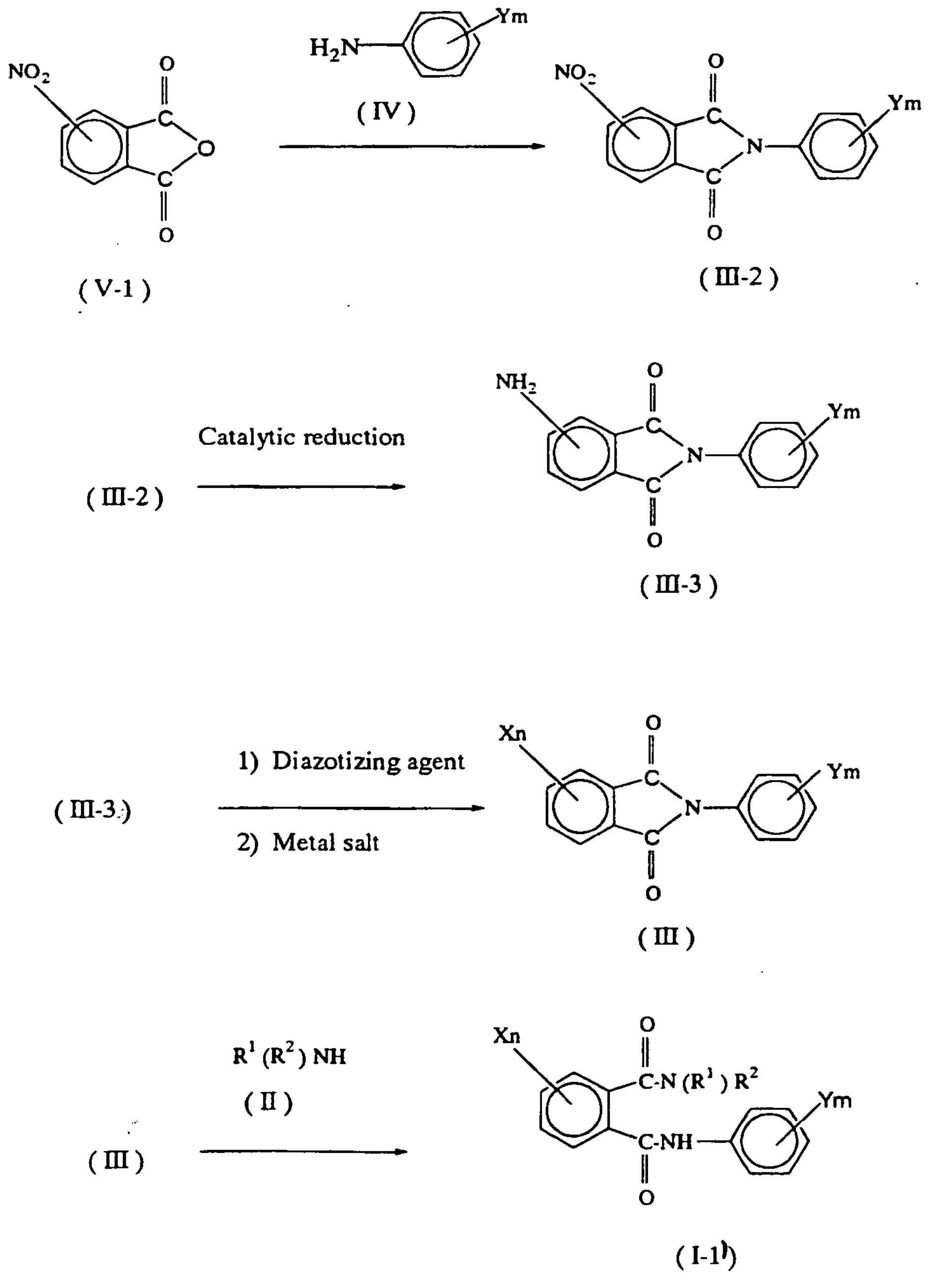


wherein $R^1$, $R^2$, X, Y, and m are as defined above and n is 2 with X being attached to the 3 and 4 position.

A phthalic anhydride of the general formula (V-1) is reacted with an aniline of the general formula (IV) in the presence of an inert solvent to obtain a phthalimide derivative of the general formula (III-2). The phthalimide derivative (III-2) is subjected to catalytic reduction with hydrogen after or without isolation to obtain a phthalimide derivative of the general formula (III-3). The phthalimide derivative (III-3) is converted to a phthalimide derivative of the general formula (III) by adding a diazotizing agent and then a metal salt after or without isolation of the phthalimide derivative (III-3). The phthalimide derivative (III) is reacted with an amine of the general formula (II) after or without being isolated, whereby a phthalic acid diamide derivative of the general formula (I-1') can be produced.

(1) General formula (V-1) → general formula (III-2)

The desired compound can be produced by this reaction in the same manner as in production process 1-(1).

(2) General formula (III-2) → general formula (III-3)

Any solvent may be used in this reaction so long as it does not markedly inhibit the progress of the reaction. There can be exemplified alcohols such as methanol, ethanol, propanol, etc.; acyclic or cyclic ethers such as diethyl ether, dioxane, tetrahydrofuran, etc., and acids such as acetic acid, etc. These inert solvents may be used alone or as a mixture thereof.

As the catalyst for catalytic reduction used in this reaction, there can be exemplified palladium carbon, Raney nickel, palladium black, platinum black, etc. The amount of the catalyst used may be properly chosen in a range of 0.1 to 10% by weight based on the weight of the phthalimide derivative of the general formula (III-2). This reaction is carried out under a hydrogen atmosphere and the hydrogen pressure may be properly chosen in a range of 1 to 10 atmospheric pressure.

As to the reaction temperature, the reaction can be carried out in a temperature range of room temperature to the reflux temperature of the inert solvent used. Although the reaction time is varied depending on the scale of reaction, the reaction temperature, etc., it may be properly chosen in a range of several minutes to 48 hours.

After completion of the reaction, the desired compound is isolated from the reaction mixture containing the desired compound by a conventional method, and if necessary, purified by recrystallization, column chromatography, etc., whereby the desired compound can be produced. The desired compound can be subjected to the subsequent reaction without isolation from the reaction mixture.

(3) General formula (III-3) → general formula (III)

In this reaction, an acidic solvent can be used as an inert solvent. The acidic solvent includes, for example, an aqueous hydrochloric acid solution, an aqueous hydrobromic acid solution, an aqueous hydroiodic acid solution, an aqueous sulfuric acid solution, acetic acid and trifluoroacetic acid. These acidic solvents may be used alone or as a mixture thereof. In addition, these acidic solvents may be used in admixture with ethers such as tetrahydrofuran, dioxane, etc.

The diazotizing agent includes, for example, sodium nitrite, nitrosyl hydrogensulfate and alkyl nitrites. The amount of the diazotizing agent used may be properly chosen in a range of equal amount to excess amount relative to the amount of the phthalimide derivative of the general formula (III-3).

As to the reaction temperature, the reaction can be carried out in a temperature range of -50°C to the reflux temperature of the inert solvent used. Although the reaction time is varied depending on the scale of reaction, the reaction temperature, etc., it may be properly chosen in a range of several minutes to 48 hours.

As the metal salt added after the production of a diazonium salt, there can be used, for example, cuprous chloride, cuprous bromide, potassium iodide, copper cyanide, potassium xanthate and sodium thiorate. The amount of the metal salt used may be properly chosen in a range of 1 equivalent to excess equivalents per equivalent of the phthalimide derivative of the general formula (III-3).

After completion of the reaction, the desired compound is isolated from the reaction mixture containing the desired compound by a conventional method, and if necessary, purified by recrystallization, column chromatography, etc., whereby the desired compound can be produced. The desired compound can be subjected to the subsequent reaction without isolation from the reaction mixture.

The reaction can be carried out according to the method described in Org. Synth., IV, 160 (1963), Org. Synth., III, 809 (1959), J. Am. Chem. Soc., 92, 3520 (1970), etc.

(4) General formula (III) → general formula (I-1')

The desired compound can be produced by this reaction in the same manner as in production process 1-(2).

Production process 4.

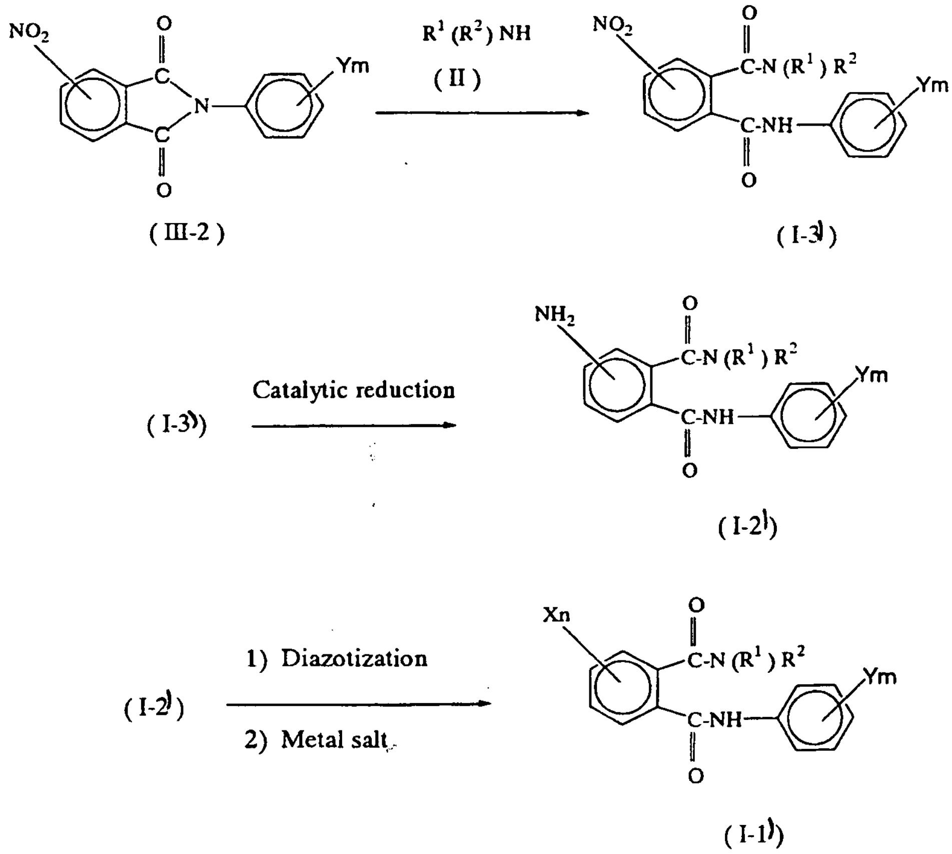


(III-2) (I-3') (I-2') (I-1')

wherein $R^1$, $R^2$, X, Y, and m are as defined above and n is 2 with X being attached to the 3 and 4 position.

A phthalimide derivative of the general formula (III-2) is reacted with an amine of the general formula (II) in the presence of an inert solvent to obtain a phthalic acid diamide derivative of the general formula (I-3'). The phthalic acid diamide derivative (I-3') is subjected to catalytic reduction with hydrogen after or without isolation to obtain a phthalic acid diamide derivative of the general formula (I-2'). A phthalic acid diamide derivative of the general formula (I-1) can be produced from the phthalic acid diamide derivative (I-2') by adding a diazotizing agent and then a metal salt after or without isolating the phthalic acid diamide derivative (I-2').

(1) General formula (III-2) → general formula (I-3')

The desired compound can be produced by this reaction in the same manner as in production process 1-(2).

(2) General formula (I-3') → general formula (I-2')

The desired compound can be produced by this reaction in the same manner as in production process 3-(2).

(3) General formula (I-2') → general formula (I-1')

The desired compound can be produced by this reaction in the same manner as in production process 3-(3).

Production process 5.

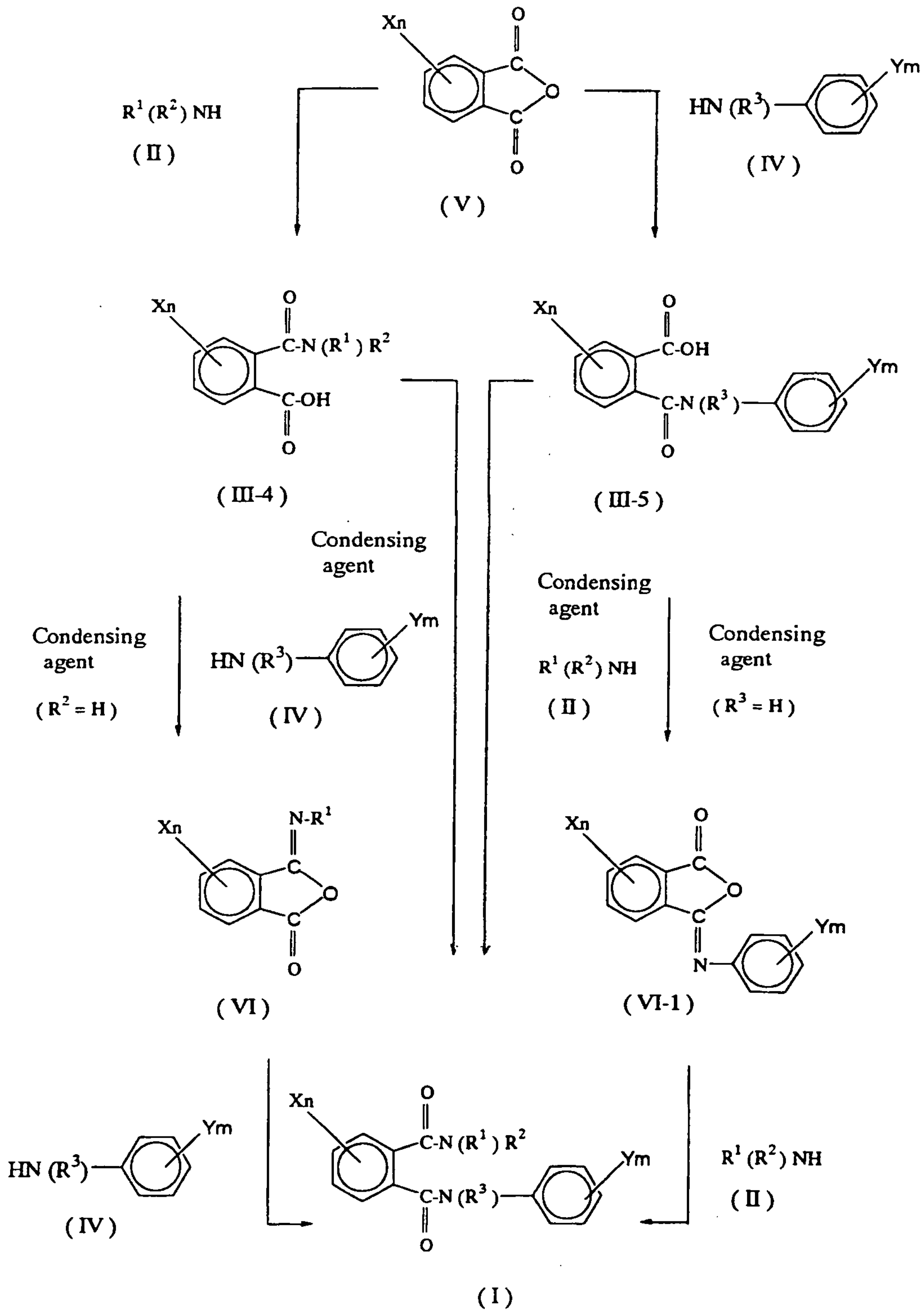


(II) (V) (IV)

(III-4) (III-5)

(IV) (II)

(VI) (VI-1)

(IV) (II)

(I)

wherein $R^1$, $R^2$, $R^3$, X, Y and m are as defined above and n is 2 with X being attached to the 3 and 4-position.

A phthalic anhydride derivative of the general formula (V) is reacted with an amine of the general formula (II) in the presence of an inert solvent to obtain a phthalamide of the general formula (III-4). The phthalamide (III-4) is treated as follows after or without isolation. When $R^2$ of the phthalamide (III-4) is a hydrogen atom, the phthalamide (III-4) is condensed into a compound of the general formula (VI) in the presence of a condensing agent, and the compound (VI) is reacted with an aniline of the general formula (IV) in the presence of an inert solvent after or without being isolated. When $R^2$ of the phthalamide (III-4) is other than a hydrogen atom, the phthalamide (III-4) is condensed with an aniline

of the general formula (IV) in the presence of a condensing agent. Thus, a phthalic acid diamide derivative of the general formula (I) can be produced.

Alternatively, a phthalic anhydride derivative of the general formula (V) is reacted with an aniline of the general formula (IV) in the presence of an inert solvent to obtain a phthalanilide of the general formula (III-5). The phthalanilide (III-5) is treated as follows after or without isolation. When $R^3$ of the phthalanilide (III-5) is a hydrogen atom, the phthalanilide (III-5) is condensed into a compound of the general formula (VI-1) in the presence of a condensing agent, and the compound (VI-1) is reacted with an amine of the general formula (II) in the presence of an inert solvent after or without being isolated. When $R^3$ of the phthalanilide (III-5) is other than a hydrogen atom, the phthalanilide (III-5) is condensed with an amine of the general formula (II) in the presence of a condensing agent. Thus, a phthalic acid diamide derivative of the general formula (I) can be produced.

(1) General formula (V) or general formula (VI-1) → general formula (111-4) or general formula (I), respectively

The desired compound can be produced by this reaction in the same manner as in production process 1-(2).

(2) General formula (III-4) or general formula (III-5) → general formula (VI) or general formula (VI-1), respectively

The desired compound can be produced by this reaction according to the method described in J. Med. Chem., 10, 982 (1967).

(3) General formula (VI) or general formula (V) → general formula (I) or general formula (III-5), respectively

The desired compound can be produced by this reaction in the same manner as in production process 1-(2).

(4) General formula (111-4) or general formula (III-5) → general formula (I)

The desired compound can be produced by reacting the phthalamide derivative of the general formula (III-4) or the general formula (III-5) with the aniline of the general formula (IV) or the amine of the general formula (II), respectively, in the presence of a condensing agent and an inert solvent. If necessary, the reaction can be carried out in the presence of a base.

The inert solvent used in the reaction includes, for example, tetrahydrofuran, diethyl ether, dioxane, chloroform and dichloromethane. As the condensing agent used in the reaction, any condensing agent may be used so long as it is used in usual amide synthesis. The condensing agent includes, for example, Mukaiyama reagent (e.g. 2-chloro-N-methylpyridinium iodide), 1,3-dicyclohexylcarbodiimide (DCC), carbonyldiimidazole (CDI) and diethyl phosphorocyanidate (DEPC). The amount of the condensing agent used may be properly chosen in a range of 1 mole to excess moles per mole of the phthalamide derivative of the general formula (III-4) or the general formula (III-5).

As the base usable in the reaction, there can be exemplified organic bases such as triethylamine, pyridine, etc. and inorganic bases such as potassium carbonate, etc. The amount of the base used may be properly chosen in a range of 1 mole to excess moles per mole of the phthalamide derivative of the general formula (III-4) or the general formula (III-5).

As to the reaction temperature, the reaction can be carried out in a temperature range of 0°C to the boiling point of the inert solvent used. Although the reaction time is varied depending on the scale of reaction, the reaction temperature, etc., it ranges from several minutes to 48 hours.

After completion of the reaction, the desired compound is isolated from the reaction solution containing the desired compound by a conventional method, and if necessary, purified by recrystallization, column chromatography, etc., whereby the desired compound can be produced.

Production process 6

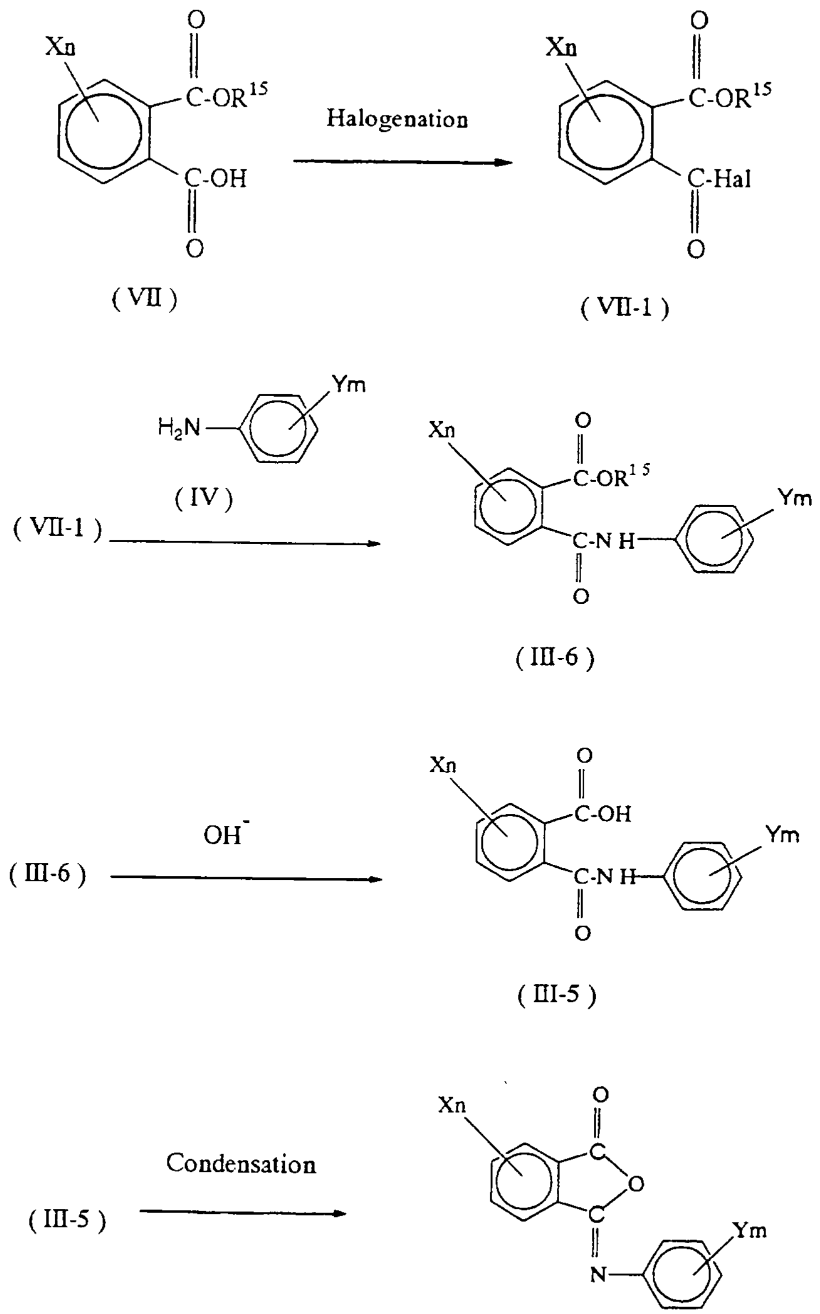


(VII) (VII-1)

(IV)

(VII-1) → (III-6)

(III-6) → (III-5)

(III-5) → (VI-1)

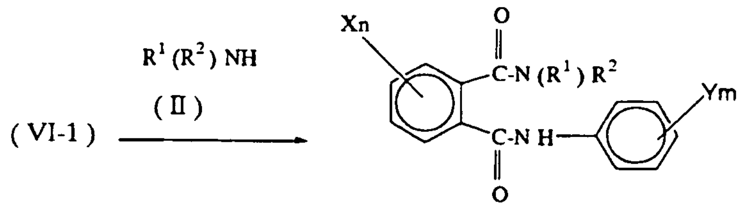


(I-1')

wherein $R^1$, $R^2$, X, Y and m are as defined above, n is 2 with X being attached to the 3 and 4-position Hal is a halogen atom, and $R^{15}$ is a $(C_1$-$C_3)$alkyl group.

A phthalic acid ester derivative of the general formula (VII) is halogenated into a phthaloyl halide of the general formula (VII-1) in the presence or absence of an inert solvent. The phthaloyl halide (VII-1) is reacted with an aniline of the general formula (IV) in the presence of an inert solvent and a base after or without being isolated, to obtain a phthalanilide of the general formula (III-6). The phthalanilide (III-6) is hydrolyzed into a phthalanilide of the general formula (III-5) in the presence or absence of an inert solvent after or without being isolated. The phthalanilide (III-5) is condensed into a phthalic anhydride derivative of the general formula (VI-1) after or without being isolated. The phthalic anhydride derivative (VI-1) is reacted with an amine of the general formula (II), whereby a phthalic acid diamide derivative of the general formula (I-1) can be produced.

(1) General formula (VII) → general formula (VII-1)

As the inert solvent usable in this reaction, any solvent may be used so long as it does not markedly inhibit the progress of the reaction. There can be exemplified aromatic hydrocarbons such as benzene, toluene, xylene, etc.; halogenated hydrocarbons such as dichloromethane, chloroform, carbon tetrachloride, etc., chlorinated aromatic hydrocarbons such as chlorobenzene, dichlorobenzene, etc.; acyclic or cyclic ethers such as diethyl ether, dioxane, tetrahydrofuran, etc., and esters such as ethyl acetate, etc. These inert solvents may be used alone or as a mixture thereof.

As the halogenating agents, there can be used, for example, thionyl chloride, phosphoryl chloride, and phosphorus trichloride. The amount of the halogenating agent used may be properly chosen in a range of 1 to 10 equivalents per equivalent of the phthalic acid ester of the general formula (VII).

As to the reaction temperature, the reaction can be carried out in a temperature range of 0°C to the reflux temperature of the inert solvent used. Although the reaction time is varied depending on the scale of reaction, the reaction temperature, etc., it may be properly chosen in a range of several minutes to 48 hours.

After completion of the reaction, the desired compound is isolated from the reaction solution containing the desired compound by a conventional method, and if necessary, purified by recrystallization, column chromatography, etc., whereby the desired compound can be produced. The desired compound can be subjected to the subsequent reaction without isolation from the reaction solution.

The phthalic acid ester of the general formula (VII) can be produced, for example, by the process described in J. Med. Chem., 31, 1466 (1988).

(2) General formula (VII-1) →general formula (III-6)

As the inert solvent used in this reaction, there may be used, for example, the inert solvents exemplified in production process 1-(1).

As the base, an inorganic base or an organic base may be used. As the inorganic base, there may be used, for example, hydroxides of alkali metals, such as sodium hydroxide, potassium hydroxide, etc. As the organic base, there may be used triethylamine, pyridine, etc. The amount of the base used may be properly chosen in a range of 0.5 to 3 equivalents per equivalent of the phthaloyl halide of the general formula (VII-1).

Since the reaction is an equimolar reaction, it is sufficient that the reactants are used in equimolar amounts, though the amount of the aniline of the general formula (IV) used may be properly chosen in a range of 0.5 to 2 equivalents per equivalent of the phthaloyl halide of the general formula (VII-1).

As to the reaction temperature, the reaction can be carried out in a temperature range of 0°C to the reflux temperature of the inert solvent used. Although the reaction time is varied depending on the scale of reaction, the reaction temperature, etc., it may be properly chosen in a range of several minutes to 48 hours.

After completion of the reaction, the desired compound is isolated from the reaction solution containing the

desired compound by a conventional method, and if necessary, purified by recrystallization, column chromatography, etc., whereby the desired compound can be produced. The desired compound can be subjected to the subsequent reaction without isolation from the reaction solution.

(3) General formula (III-6) → general formula (III-5)

As the inert solvent usable in this reaction, there may be used water, alcohols (e.g. methanol, ethanol and propanol) as water-soluble solvents, and mixed solvents of water and a water-soluble solvent.

As the base used for the hydrolysis, there may be used, for example, hydroxides of alkali metals, such as sodium hydroxide, potassium hydroxide, etc. The amount of the base used may be properly chosen in a range of 1 to 10 equivalents per equivalent of the phthalanilide of the general formula (III-6).

As to the reaction temperature, the reaction can be carried out in a temperature range of 0°C to the reflux temperature of the inert solvent used. Although the reaction time is varied depending on the scale of reaction, the reaction temperature, etc., it may be properly chosen in a range of several minutes to 48 hours.

After completion of the reaction, the desired compound is isolated from the reaction solution containing the desired compound by a conventional method, and if necessary, purified by recrystallization, column chromatography, etc., whereby the desired compound can be produced. The desired compound can be subjected to the subsequent reaction without isolation from the reaction solution.

(4) General formula (III-5) → general formula (VI-1)

The desired compound can be produced by this reaction according to production process 5-(2).

(5) General formula (VI-1) → general formula (I-1')

The desired compound can be produced by this reaction according to production process 1-(2).

Production process 7.

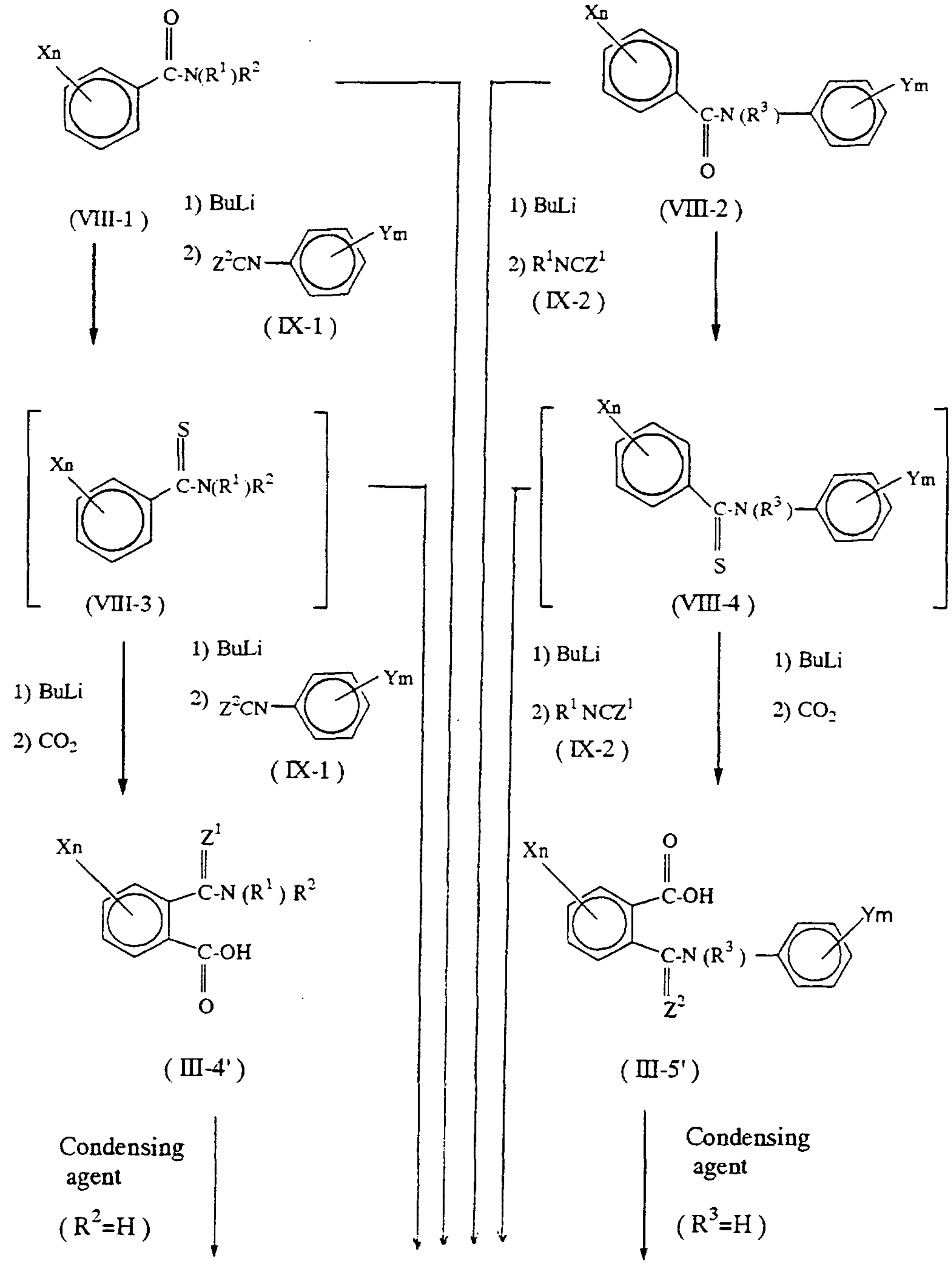

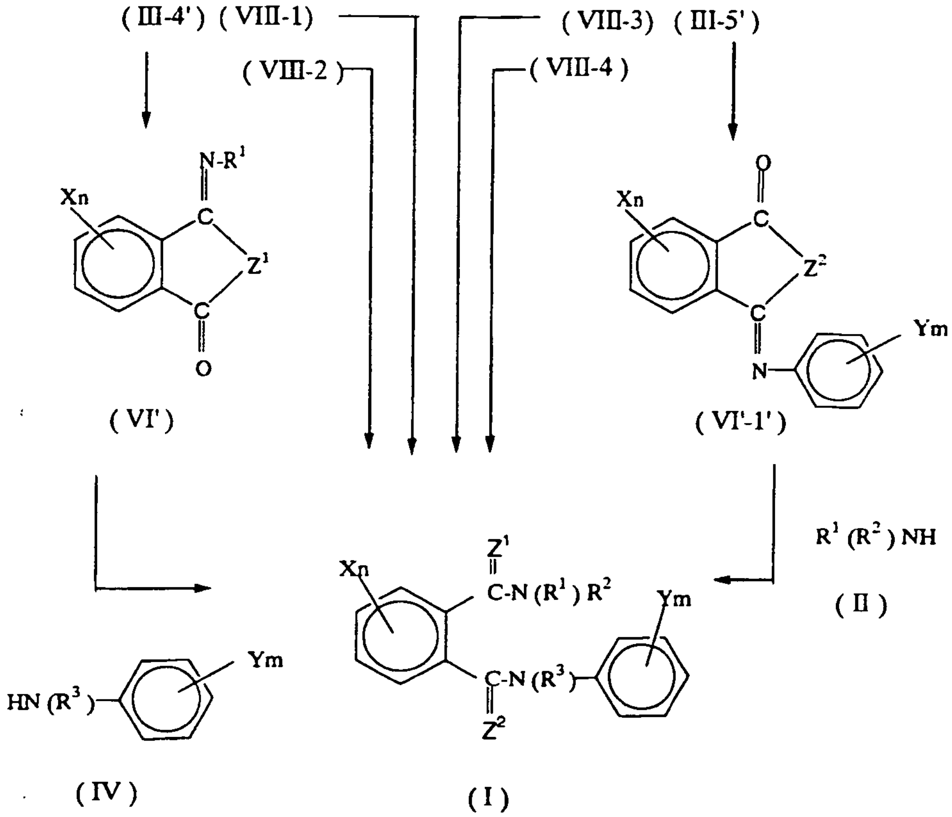

wherein $R^1$, $R^2$, $R^3$, X, Y, m, $Z^1$ and $Z^2$ are as defined above and n is 2 with X being attached to the 3 and 4-position.

A benzamide derivative of the general formula (VIII-1) or the general formula (VIII-2) or a thiobenzamide derivative of the general formula (VIII-3) or the general formula (VIII-4) obtained by thiocarbonylation of the benzamide derivative of the general formula (VIII-1) or the general formula (VIII-2), respectively, is subjected to ortho-metallation by using a metal reagent such as butyllithium. The compound thus obtained is directly reacted with an isocyanate or isothiocyanate derivative of the general formula (IX-1) or (IX-2), or the compound is reacted with carbon dioxide to obtain a phthalamide derivative of the general formula (III-4') or the general formula (III-5'), which is treated in the same manner as in production processes 5-(1) to 5-(4). Thus, a phthalic acid diamide derivative of the general formula (I) can be produced.

(1) General formula (VIII-1) or general formula (VIII-2) → general formula (VIII-3) or general formula (VIII-4) respectively

The desired compound can be produced by this reaction according to the method described in J. Org. Chem., 46, 3558 (1981).

(2) General formula (VIII-1), general formula (VIII-2), general formula (VIII-3) or general formula (VIII-4) → general formula (I)

In this step, the benzamide derivative of the general formula (VIII-1) or the general formula (VIII-2) or the thiobenzamide derivative of the general formula (VIII-3) or the general formula (VIII-4) obtained by thiocarbonylation of the benzamide derivative of the general formula (VIII-1) or the general formula (VIII-2), respectively, is subjected to ortho-lithiation according to the method described in J. Org. Chem., 29, 853 (1964). The compound thus obtained is reacted with the isocyanate or isothiocyanate derivative of the general formula (IX-1) or (IX-2) at -80°C to room temperature, whereby the desired compound can be produced.

After completion of the reaction, the desired compound is isolated from the reaction solution containing the desired compound by a conventional method, and if necessary, purified by recrystallization, column chromatography, etc., whereby the desired compound can be obtained.

(3) General formula (VIII-1), general formula (VIII-2), general formula (VIII-3) or general formula (VIII-4) → general formula (III-4') or the general formula (III-5')

In this step, the desired compound can be produced by carrying out the same ortho-lithiation as in the above step (2) and introducing carbon dioxide into the ortho-lithiation product at -80°C to room temperature.

After completion of the reaction, the desired compound is isolated from the reaction solution containing the desired compound by a conventional method, and if necessary, purified by recrystallization, column chromatography, etc., whereby the desired compound can be obtained.

(4) General formula (III-4') or general formula (III-5') → general formula (I)

In this step, the desired compound can be produced in the same manner as in production process 1-(2) or 5-(4).

Tables 1 and 2 show typical examples of the phthalic acid diamide derivative of the general formula (I) used as the active ingredient of the agricultural and horticultural insecticide of the present invention, but the examples are not intended in any way to limit the scope of the present invention.

General formula (I):

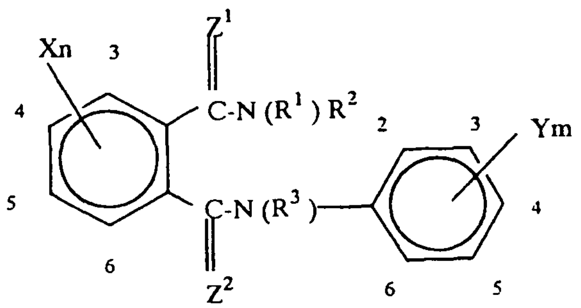

(I)

Table 1 ($Z^1$, $Z^2$ = O)

| No | $R^1$ | $R_2$ | $R_3$ | Xn | Ym | Physical Properties (melting point: °C |
|---|---|---|---|---|---|---|
| *1 | H | H | H | $3-NO_2$ | $2-CH_3-5-Cl$ | 173-175 |
| *2 | $CH_3$ | H | H | H | $4-CF_3$ | 129-131 |
| *3 | $CH_3$ | H | H | $3-NO_2$ | $2-CH_3-5-Cl$ | 169-171 |
| *4 | $CH_3$ | H | H | $3-NO_2$ | $2-CH_3-4-OCHF_2$ | 167-169 |
| *10 | $C_2H_5$ | H | H | $3-NO_2$ | $2-CH_3-5-Cl$ | 175-177 |
| *11 | $C_2H_5$ | H | H | $3-NO_2$ | $2-CH_3-4-OCHF_2$ | 207-208 |
| *12 | $C_2H_5$ | $C_2H_5$ | H | H | $4-CF_3$ | 148-150 |
| *13 | $C_2H_5$ | $C_2H_5$ | H | $3-NO_2$ | $2-CH_3-5-Cl$ | 175-177 |
| *17 | $n-C_3H_7$ | H | H | $3-NO_2$ | $2-CH_3-4-OCHF_2$ | 184-186 |
| *18 | $n-C_3H_7$ | H | H | $3-NO_2$ | $2-CH_3-5-Cl$ | 187-189 |
| *22 | $i-C_3H_7$ | H | H | H | $4-NO_2$ | 139-141 |
| *23 | $i-C_3H_7$ | H | H | H | 4-F | 199-201 |
| *24 | $i-C_3H_7$ | H | H | H | $2-CH_3$ | 191-193 |
| *26 | $i-C_3H_7$ | H | H | H | $3-CF_3$ | 174-176 |
| *27 | $i-C_3H_7$ | H | H | H | $4-CF_2CF_2CF_3$ | 237-238 |

(continued)

| No | $R^1$ | $R_2$ | $R_3$ | Xn | Ym | Physical Properties (melting point: °C |
|---|---|---|---|---|---|---|
| *34 | i-$C_3H_7$ | H | H | H | 4-$SCF_2CHF_2$ | 169-170 |
| *42 | i-$C_3H_7$ | H | H | H | 2,4-$Cl_2$ | 162-164 |
| *43 | i-$C_3H_7$ | H | H | H | 3,4-$F_2$ | 172-174 |
| *47 | i-$C_3H_7$ | H | H | H | 2-Cl-4-$OCF_3$ | 151-153 |
| *71 | i-$C_3H_7$ | H | H | H | 2-$CH_3$-4-$COOCH_3$ | 163-165 |
| *76 | i-$C_3H_7$ | H | H | H | 2-$CH_3$-4-(4-Cl-Ph-$CH_2$O) | 188-189 |
| *77 | i-$C_3H_7$ | H | H | H | 2-$CH_3$-4-(4-Cl-PhS) | 181-182 |
| *79 | i-$C_3H_7$ | H | H | H | 2-$CH_3$-4-(3-Cl-5-$CF_3$-2-Pyi-O) | 184-185 |
| *98 | i-$C_3H_7$ | H | H | 3-Cl | 4-$SCHF_2$ | 220-222 |
| *102 | i-$C_3H_7$ | H | H | 3-Cl | 4-$SCF_2CHF_2$ | 198-200 |
| *103 | i-$C_3H_7$ | H | H | 3-Cl | 4-$SCF_2CBrF_2$ | 201-203 |
| *109 | i-$C_3H_7$ | H | H | 3-Cl | 4-$COCH_3$ | 217-219 |
| *111 | i-$C_3H_7$ | H | H | 3-Cl | 2,3-$Cl_2$ | 168-169 |
| *115 | i-$C_3H_7$ | H | H | 3-Cl | 2-F-4-Cl | 181-182 |
| *116 | i-$C_3H_7$ | H | H | 3-Cl | 2,3,4-$F_3$ | 174-176 |
| *120 | i-$C_3H_7$ | H | H | 3-Cl | 2-$CH_3$-5-Cl | 183-185 |
| *128 | i-$C_3H_7$ | H | H | 3-Cl | 2-$CH_3$-4-$CF_2CF_3$ | 199-200 |
| *147 | i-$C_3H_7$ | H | H | 3-Cl | 2-$CH_3$-4-$OCH_2CF_2CHF_2$ | 207-208 |
| *153 | i-$C_3H_7$ | H | H | 3-Cl | 2-$CH_3$-4-$SC_3H_7$-i | 189-191 |
| *161 | i-$C_3H_7$ | H | H | 3-Cl | 2-$CH_3$-4-P=O $(OC_2H_5)_2$ | 71-73 |
| *165 | i-$C_3H_7$ | H | H | 3-Cl | 3-N=C($CF_3$)-O-4 | 248-250 |
| *169 | i-$C_3H_7$ | H | H | 4-Cl | 4-F | 213-215 |
| *170 | i-$C_3H_7$ | H | H | 4-Cl | 2-$CH_3$ | 208-210 |
| *171 | i-$C_3H_7$ | H | H | 4-Cl | 3-$CF_3$ | 196-198 |
| *172 | i-$C_3H_7$ | H | H | 4-Cl | 4-$OCF_3$ | 192-194 |
| *173 | i-$C_3H_7$ | H | H | 4-Cl | 2,4-$Cl_2$ | 174-176 |
| *176 | i-$C_3H_7$ | H | H | 4-Cl | 2-$CH_3$-3-Cl | 203-205 |
| *186 | i-$C_3H_7$ | H | H | 5-Cl | 2,3-$Cl_2$ | 167-169 |
| *187 | i-$C_3H_7$ | H | H | 5-Cl | 2,4-$Cl_2$ | 165-167 |
| *188 | i-$C_3H_7$ | H | H | 5-Cl | 3,4-$F_2$ | 207-209 |
| *193 | i-$C_3H_7$ | H | H | 5-Cl | 2-$CH_3$-4-$OCHF_2$ | 188-189 |
| *196 | i-$C_3H_7$ | H | H | 6-Cl | 4-$C_4H_9$-t | 235-237 |
| *198 | i-$C_3H_7$ | H | H | 6-Cl | 4-$CF(CF_3)_2$ | 209-211 |
| *210 | i-$C_3H_7$ | H | H | 6-Cl | 2,4-$F_2$ | 196-198 |
| *212 | i-$C_3H_7$ | H | H | 6-Cl | 2-F-4-Cl | 184-186 |
| *215 | i-$C_3H_7$ | H | H | 6-Cl | 2-$CH_3$-5-Cl | 204-206 |
| *218 | i-$C_3H_7$ | H | H | 6-Cl | 2-$CH_3$-4-$OCH_3$ | 195-197 |
| *219 | i-$C_3H_7$ | H | H | 6-Cl | 2,3-$(CH_3)_2$-4-$OCH_3$ | 242-244 |

(continued)

| No | $R^1$ | $R_2$ | $R_3$ | Xn | Ym | Physical Properties (melting point: °C |
|---|---|---|---|---|---|---|
| *229 | i-$C_3H_7$ | H | H | 6-Cl | 2-$CH_3$-4-$OCF_2CBrF_2$ | 214-215 |
| *230 | i-$C_3H_7$ | H | H | 6-Cl | 2-$CH_3$-4-$OCH_2CF_2CHF_2$ | 212-213 |
| *244 | i-$C_3H_7$ | H | H | 6-Cl | 2-$CH_3$-4-$OCF_2CHFOCF_3$ | 210-212 |
| *245 | i-$C_3H_7$ | H | H | 6-Cl | 2-$CH_3$-4-$OCHF_2$-5-Cl | 234-235 |
| *246 | i-$C_3H_7$ | H | H | 6-Cl | 2-$CH_3$-4-$OCF_2CHF_2$-5-Cl | 230-232 |
| *248 | i-$C_3H_7$ | H | H | 6-Cl | 2-$CH_3$-4-($F_5$-PhO) | 243-245 |
| *249 | i-$C_3H_7$ | H | H | 6-Cl | 2-$CH_3$-4-(5-$CF_3$-2-Pyi-O) | 116-120 |
| *250 | i-$C_3H_7$ | H | H | 6-Cl | 2-$CH_3$-4-(3-Cl-5-$CF_3$-2-Pyi-O) | 219-221 |
| *253 | i-$C_3H_7$ | H | H | 6-Cl | 2-$CF_3$-4-$OCHF_2$ | 234-236 |
| *259 | i-$C_3H_7$ | H | H | 6-Cl | 2-$CF_3$-4-Cl | 234-235 |
| *261 | i-$C_3H_7$ | H | H | 3-Br | 4-$CF_3$ | 221-223 |
| *262 | i-$C_3H_7$ | H | H | 3-Br | 4-$OCF_3$ | 208-210 |
| *264 | i-$C_3H_7$ | H | H | 3-Br | 2,4-$(CH_3)_2$ 2 | 223-224 |
| *273 | i-$C_3H_7$ | H | H | 3-Br | 2,3-$(CH_3)_2$-4-Br | 200-202 |
| *275 | i-$C_3H_7$ | H | H | 3-Br | 2-$CH_3$-4-F | 223-224 |
| *285 | i-$C_3H_7$ | H | H | 3-Br | 2,3-$(CH_3)_2$-4-$OCH_3$ | 206-208 |
| *295 | i-$C_3H_7$ | H | H | 3-Br | 2,3-$(CH_3)_2$-4-$OCHF_2$ | 219-220 |
| *301 | i-$C_3H_7$ | H | H | 6-Br | 4-$CF_3$ | 190-192 |
| *302 | i-$C_3H_7$ | H | H | 6-Br | 4-$OCF_3$ | 210-212 |
| *303 | i-$C_3H_7$ | H | H | 6-Br | 2,3-$(CH_3)_2$ | 250-252 |
| *307 | i-$C_3H_7$ | H | H | 6-Br | 2-$CH_3$-5-Cl | 194-196 |
| *311 | i-$C_3H_7$ | H | H | 6-Br | 2-$CH_3$-4-Br | 227-228 |
| *319 | i-$C_3H_7$ | H | H | 6-Br | 2-$CH_3$-4-$OCH_2CF_2CHF_2$ | 209-211 |
| *320 | i-$C_3H_7$ | H | H | 6-Br | 2,4-$(CH_3)_2$-3-$OCHF_2$ | 247-249 |
| *323 | i-$C_3H_7$ | H | H | 6-Br | 2-Cl-4-$OCF_3$ | 182-183 |
| *333 | i-$C_3H_7$ | H | H | 6-Br | 2-$CH_3$-4-$SCH_3$ | 215-217 |
| *334 | i-$C_3H_7$ | H | H | 6-Br | 2-$CH_3$-4-(3-$CF_3$-PhO) | 194-195 |
| *336 | i-$C_3H_7$ | H | H | 6-Br | 2-$CH_3$-4-(3-Cl-5-$CF_3$-2-Pyi-O) | 234-236 |
| 338 | i-$C_3H_7$ | H | H | 3,4-$Br_2$ | 2-$CH_3$-4-$OCHF_2$ | 196-197 |
| 339 | i-$C_3H_7$ | H | H | 3,4-$Br_2$ | 2-$CH_3$-4-Cl | 199-201 |
| *346 | i-$C_3H_7$ | H | H | 3-I | 4-Br | 251-253 |
| *347 | i-$C_3H_7$ | H | H | 3-I | 4-I | 231-233 |
| *355 | i-$C_3H_7$ | H | H | 3-I | 4-$SCH_2CF_3$ | 195-197 |
| *360 | i-$C_3H_7$ | H | H | 3-1 | 3,4-$F_2$ | 227-229 |

(continued)

| No | $R^1$ | $R_2$ | $R_3$ | Xn | Ym | Physical Properties (melting point: °C |
|---|---|---|---|---|---|---|
| *364 | i-$C_3H_7$ | H | H | 3-I | 2,4-$(CH_3)_2$-3-Cl | 226-228 |
| *378 | i-$C_3H_7$ | H | H | 3-I | 2,3-$(CH_3)_2$-4-$OCH_3$ | 220-222 |
| *384 | i-$C_3H_7$ | H | H | 3-1 | 3-$OCH_3$-4-$OCHF_2$ | 196-198 |
| *400 | i-$C_3H_7$ | H | H | 3-I | 2-$CH_3$-4-(3-Cl-5-$CF_3$-2-Pyi-0) | 212-214 |
| *412 | i-$C_3H_7$ | H | H | 6-1 | 4-Br | 270-272 |
| *413 | i-$C_3H_7$ | H | H | 6-I | 4-I | 242-244 |
| *420 | i-$C_3H_7$ | H | H | 6-I | 4-$SCH_2CF_3$ | 217-219 |
| *427 | i-$C_3H_7$ | H | H | 6-1 | 2-$CH_3$-3-Cl | 252-254 |
| *429 | i-$C_3H_7$ | H | H | 6-I | 2,4-$(CH_3)_2$-3-Cl | 260-262 |
| *430 | i-$C_3H_7$ | H | H | 6-I | 2-$CH_3$-4-Br | 241-243 |
| *437 | i-$C_3H_7$ | H | H | 6-I | 2-$CH_3$-4-$CF_2CF_3$ | 203-205 |
| *440 | i-$C_3H_7$ | H | H | 6-I | 2-$CH_3$-4-$(CF_2)_3CF_3$ | 168-170 |
| *443 | i-$C_3H_7$ | H | H | 6-I | 2-$CH_3$-4-$OCH_2CF_3$ | 233-234 |
| *447 | i-$C_3H_7$ | H | H | 6-I | 2-$CH_3$-4-$OCH_2CF_2CHF_2$ | 235-237 |
| *450 | i-$C_3H_7$ | H | H | 6-I | 3-$OCH_3$-4-$OCHF_2$ | 215-217 |
| *453 | i-$C_3H_7$ | H | H | 6-I | 2-$CH_3$-4-$OCF_2CHF_2$ | 216-218 |
| *469 | i-$C_3H_7$ | H | H | 6-1 | 2-$CH_3$-4-$OCHF_2$ | 226-228 |
| *470 | i-$C_3H_7$ | H | H | 6-I | 2-$CH_3$-4-$OCHF_2$-5-Cl | 239-240 |
| *475 | i-$C_3H_7$ | H | H | 3-F | 4-$OCF_3$ | 197-199 |
| *477 | i-$C_3H_7$ | H | H | 3-F | 4-$SCHF_2$ | 190-192 |
| *515 | i-$C_3H_7$ | H | H | 3-F | 2-$CH_3$-4-$OCF_2CHFOCF_3$ | 148-150 |
| *522 | i-$C_3H_7$ | H | H | 3-F | 2-$CH_3$-4-(4-Cl-PhS) | 176-178 |
| *529 | i-$C_3H_7$ | H | H | 4-F | 2-$CH_3$-4-Cl | 217-218 |
| *530 | i-$C_3H_7$ | H | H | 4-F | 2-$CH_3$-5-Cl | 202-203 |
| *547 | i-$C_3H_7$ | H | H | 6-F | 2-$CH_3$-4-I | 213-215 |
| *549 | i-$C_3H_7$ | H | H | 6-F | 2-$CH_3$-5-F | 181-183 |
| *550 | i-$C_3H_7$ | H | H | 6-F | 2-Cl-4-$CF_3$ | 190-191 |
| *553 | i-$C_3H_7$ | H | H | 6-F | 2-$CH_3$-4-$OCF_2CCl_2F$ | 214-215 |
| *557 | i-$C_3H_7$ | H | H | 6-F | 2-$CH_3$-4-$(CF_2)_3CF_3$ | 157-159 |
| *561 | i-$C_3H_7$ | H | H | 6-F | 2-Cl-4-$CF(CF_3)_2$ | 241-243 |
| *567 | i-$C_3H_7$ | H | H | 6-F | 2-$CH_3$-4-$OCF_2CHClF$ | 175-176 |
| *568 | i-$C_3H_7$ | H | H | 6-F | 2-$CH_3$-4-$OCF_2CHFCF_3$ | 176-178 |

(continued)

| No | $R^1$ | $R_2$ | $R_3$ | Xn | Ym | Physical Properties (melting point: °C |
|---|---|---|---|---|---|---|
| *571 | $i-C_3H_7$ | H | H | 6-F | $2-CH_3-4-OCHF_2-5-Cl$ | 209-211 |
| *576 | $i-C_3H_7$ | H | H | 6-F | $2-CH_3-4-(5-CF_3-2-Pyi-0)$ | 206-207 |
| *584 | $i-C_3H_7$ | H | H | $3-NO_2$ | $2,3-Cl_2$ | 201-203 |
| *586 | $i-C_3H_7$ | H | H | $3-NO_2$ | 2-Cl | 190-192 |
| *591 | $i-C_3H_7$ | H | H | $3-NO_2$ | 4-Br | 205-207 |
| *596 | $i-C_3H_7$ | H | H | $3-NO_2$ | $4-NO_2$ | 201-203 |
| *599 | $i-C_3H_7$ | H | H | $3-NO_2$ | $2-CH_3$ | 227-228 |
| *603 | $i-C_3H_7$ | H | H | $3-NO_2$ | $2-C_3H_7-i$ | 190-192 |
| *604 | $i-C_3H_7$ | H | H | $3-NO_2$ | $4-C_3H_7-i$ | 221-223 |
| *605 | $i-C_3H_7$ | H | H | $3-NO_2$ | $4-C_4H_9-n$ | 193-195 |
| *606 | $i-C_3H_7$ | H | H | $3-NO_2$ | $4-CF_3$ | 192-194 |
| *610 | $i-C_3H_7$ | H | H | $3-NO_2$ | $4-CF(CF_3)_2$ | 243-244 |
| *612 | $i-C_3H_7$ | H | H | $3-NO_2$ | $2-OCH_3$ | 172-174 |
| *616 | $i-C_3H_7$ | H | H | $3-NO_2$ | $3-OCHF_2$ | 188-190 |
| *625 | $i-C_3H_7$ | H | H | $3-NO_2$ | $4-SCHF_2$ | 183-185 |
| *633 | $i-C_3H_7$ | H | H | $3-NO_2$ | $2,4-Cl_2$ | 202-204 |
| *634 | $i-C_3H_7$ | H | H | $3-NO_2$ | $2,5-Cl_2$ | 230-232 |
| *635 | $i-C_3H_7$ | H | H | $3-NO_2$ | $2,6-Cl_2$ | 210-212 |
| *636 | $i-C_3H_7$ | H | H | $3-NO_2$ | $3,4-Cl_2$ | 227-229 |
| *641 | $i-C_3H_7$ | H | H | $3-NO_2$ | $2,6-F_2$ | 173-175 |
| *646 | $i-C_3H_7$ | H | H | $3-NO_2$ | $2,3,4,5,6-F_5$ | 192-194 |
| *648 | $i-C_3H_7$ | H | H | $3-NO_2$ | $2,4-(CH_3)_2$ | 201-203 |
| *649 | $i-C_3H_7$ | H | H | $3-NO_2$ | $2,5-(CH_3)_2$ | 221-223 |
| *654 | $i-C_3H_7$ | H | H | $3-NO_2$ | $3,5-(CF_3)_2$ | 225-227 |
| *657 | $i-C_3H_7$ | H | H | $3-NO_2$ | $2-F-4-Cl-5-CH_3$ | 193-195 |
| *661 | $i-C_3H_7$ | H | H | $3-NO_2$ | $2-Cl-5-CF_3$ | 193-195 |
| *663 | $i-C_3H_7$ | H | H | $3-NO_2$ | $2-CH_3-3-Cl$ | 198-200 |
| *666 | $i-C_3H_7$ | H | H | $3-NO_2$ | $2-CH_3-6-Cl$ | 248-250 |
| *669 | $i-C_3H_7$ | H | H | $3-NO_2$ | $2,3-(CH_3)_2-4-Cl$ | 226-228 |
| *670 | $i-C_3H_7$ | H | H | $3-NO_2$ | $2,4-(CH_3)_2-3-Cl$ | 203-205 |
| *671 | $i-C_3H_7$ | H | H | $3-NO_2$ | $2-CH_3-4-Br$ | 214-216 |
| *673 | $i-C_3H_7$ | H | H | $3-NO_2$ | $2-CH_3-4-I$ | 227-227 |
| *674 | $i-C_3H_7$ | H | H | $3-NO_2$ | $2-CH_3-3-F$ | 199-201 |
| *676 | $i-C_3H_7$ | H | H | $3-NO_2$ | $2-CH_3-5-F$ | 213-215 |
| *680 | $i-C_3H_7$ | H | H | $3-NO_2$ | $3-CH_3-4-Br$ | 199-201 |
| *687 | $i-C_3H_7$ | H | H | $3-NO_2$ | $2-CH_3-3-OCH_3$ | 198-200 |
| *689 | $i-C_3H_7$ | H | H | $3-NO_2$ | $2,3-(CH_3)_2-4-OCH_3$ | 253-255 |
| *691 | $i-C_3H_7$ | H | H | $3-NO_2$ | $3-CF_3-5-OCH_3$ | 214-216 |
| *692 | $i-C_3H_7$ | H | H | $3-NO_2$ | $2-CF_3-4-OCHF_2$ | 201-203 |
| *699 | $i-C_3H_7$ | H | H | $3-NO_2$ | $2-CH_3-4-OCH_2CF_2CHF_2$ | 199-200 |
| *705 | $i-C_3H_7$ | H | H | $3-NO_2$ | $2-Br-4-OCF_3$ | 202-203 |
| *708 | $i-C_3H_7$ | H | H | $3-NO_2$ | $2-CH_3-5-NO_2$ | 197-199 |
| *710 | $i-C_3H_7$ | H | H | $3-NO_2$ | $2-CH_3-4-OCF_3$ | 184-186 |

(continued)

| No | $R^1$ | $R_2$ | $R_3$ | Xn | Ym | Physical Properties (melting point: °C |
|---|---|---|---|---|---|---|
| *711 | i-$C_3H_7$ | H | H | 3-$NO_2$ | 2-$CH_3$-4-$OCBrF_2$ | 217-218 |
| *713 | i-$C_3H_7$ | H | H | 3-$NO_2$ | 2-$CH_3$-3-$OCF_2CHClF$ | 164-166 |
| *719 | i-$C_3H_7$ | H | H | 3-$NO_2$ | 2-$CH_3$-4-$SCH_3$ | 214-216 |
| *723 | i-$C_3H_7$ | H | H | 3-$NO_2$ | 2-$CH_3$-4-N $(CH_3)_2$ | 215-217 |
| *735 | i-$C_3H_7$ | H | H | 3-$NO_2$ | 2-$CH_2CH_2CH_2$-3 | 226-228 |
| *738 | i-$C_3H_7$ | H | H | 5-$NO_2$ | 2-CHa-5-Cl | 226-228 |
| *743 | i-$C_3H_7$ | H | H | 6-$NO_2$ | 2-$CH_3$-4-$OCF_2CHFCF_3$ | 252-253 |
| *744 | i-$C_3H_7$ | H | H | 3-CN | 2-$CH_3$-4-Cl | 162-164 |
| *745 | i-$C_3H_7$ | H | H | 6-CN | 2-$CH_3$-4-Cl | Crystals |
| *753 | i-$C_3H_7$ | H | H | 6-$C_2H_5$ | 2-$CH_3$-4-Cl | 180-182 |
| *759 | i-$C_3H_7$ | H | H | 3-$CF_3$ | 2-$CH_3$-4-$OCHF_2$ | 212-213 |
| *767 | i-$C_3H_7$ | H | H | 3-$OCH_3$ | 4-$OCF_3$ | 178-180 |
| *769 | i-$C_3H_7$ | H | H | 6-$OCH_3$ | 4-$OCF_3$ | 189-190 |
| *771 | i-$C_3H_7$ | H | H | 6-$OCH_3$ | 2-$CH_3$-4-Br | 195-197 |
| *776 | i-$C_3H_7$ | H | H | 5-$OCHF_2$ | 2-$CH_3$-5-Cl | 173-175 |
| *777 | i-$C_3H_7$ | H | H | 6-$OCHF_2$ | 2-$CH_3$-4-Cl | 224-226 |
| *788 | i-$C_3H_7$ | H | H | 3-$SC_3H_7$-i | 2-$CH_3$-4-Cl | 183-184 |
| *797 | i-$C_3H_7$ | H | H | 3-$SCH_2CF_3$ | 2-$CH_3$-4-$OCHF_2$ | 184-186 |
| *800 | i-$C_3H_7$ | H | H | 6-$SOCH_2CF_3$ | 2-$CH_3$-4-$OCHF_2$ | 238-240 |
| *801 | i-$C_3H_7$ | H | H | 6-C≡CH | 2-$CH_3$-4-Cl | 253-255 |
| *802 | i-$C_3H_7$ | H | H | 6-$COOCH_3$ | 2-$CH_3$-4-Cl | 149-151 |
| 819 | i-$C_3H_7$ | H | H | 3-CH=CH-CH=CH-4 | 2-$CH_3$-4-$OCHF_2$ | 158-160 |
| 821 | i-$C_3H_7$ | H | H | 3-CH=CH-CH=CH-4 | 2-$CH_3$-5-Cl | 156-158 |
| *822 | i-$C_3H_7$ | H | H | 4-CH=CH-CH=CH-5 | 2-$CH_3$-5-Cl | 229-231 |
| *830 | n-$C_4H_9$ | H | H | 3-$NO_2$ | 2-$CH_3$-5-Cl | 172-174 |
| *831 | i-$C_4H_9$ | H | H | 3-$NO_2$ | 2-$CH_3$-5-Cl | 186-188 |
| *833 | i-$C_4H_9$ | H | H | H | 4-$CF_3$ | 149-151 |
| *838 | s-$C_4H_9$ | H | H | 3-$NO_2$ | 2-$CH_3$-5-Cl | 205-207 |
| *839 | s-$C_4H_9$ | H | H | 3-$NO_2$ | 2-$CH_3$-4-$OCHF_2$ | 228-229 |
| *840 | t-$C_4H_9$ | H | H | H | H | 237-239 |
| *841 | t-$C_4H_9$ | H | H | H | 2-$CH_3$-5-Cl | 200-202 |
| *842 | t-$C_4H_9$ | H | H | 3-$NO_2$ | 2-$CH_3$-5-Cl | 256-258 |
| *847 | n-$C_8H_{17}$ | H | H | 3-$NO_2$ | 2-$CH_3$-5-Cl | 164-166 |
| *851 | c-$C_3H_5$ | H | H | 3-$NO_2$ | 2-$CH_3$-5-Cl | 194-196 |
| *852 | c-$C_3H_5$ | H | H | 3-$NO_2$ | 2-$CH_3$-4-$OCHF_2$ | 191-192 |
| *856 | c-$C_5H_9$ | H | H | 3-$NO_2$ | 2-$CH_3$-4-$OCHF_2$ | 219-220 |
| *858 | c-$C_5H_9$ | H | H | 3-$NO_2$ | 2-$CH_3$-5-Cl | 200-202 |
| *859 | C-$C_6H_{11}$ | H | H | 3-$NO_2$ | 2-$CH_3$-5-Cl | 225-227 |
| *862 | $CH_2CH_2F$ | H | H | 3-$NO_2$ | 2-$CH_3$-5-F | 179-181 |
| *866 | $CH_2CH=CH_2$ | H | H | 3-$NO_2$ | 2-$CH_3$-5-Cl | 175-177 |

(continued)

| No | $R^1$ | $R_2$ | $R_3$ | Xn | Ym | Physical Properties (melting point: °C |
|---|---|---|---|---|---|---|
| *867 | $CH_2CH{=}CH_2$ | H | H | 3-$NO_2$ | 2-$CH_3$-4-$OCHF_2$ | 194-195 |
| *869 | $CH_2C{\equiv}CH$ | H | H | 3-$NO_2$ | 2-$CH_3$-5-Cl | 191-193 |
| *872 | $CH_2CH_2OCH_3$ | H | H | 3-$NO_2$ | 2-$CH_3$-4-$OCHF_2$ | 165-167 |
| *874 | $CH(CH_3)CH_2OCH_3$ | H | H | 3-$NO_2$ | 2-$CH_3$-4-$OCHF_2$ | 153-155 |
| *878 | $CH(CH_3)$-Ph | H | H | 3-$NO_2$ | 2-$CH_3$-5-Cl | 168-170 |
| *880 | $CH_2CH_2O$-(2,4-$(CH_3)_2$-Ph) | H | H | 3-$NO_2$ | 2-$CH_3$-5-Cl | 126-128 |
| *881 | -$CH_2CH_2CH_2CH_2$- | | H | H | 4-$CF_3$ | 170-171 |
| *888 | $CH_2$-3-Pyi | H | H | 3-$NO_2$ | 2-$CH_3$-4-Br | 180-182 |
| *901 | i-$C_3H_7$ | H | H | 3-F | 2-F-4-O-(4-$CF_3$-2-Cl-Ph) | 137-139 |
| *906 | i-$C_3H_7$ | H | H | 6-F | 2-$CH_3$-4-$SCF_2CF_3$ | 162-164 |
| *908 | i-$C_3H_7$ | H | H | 6-Cl | 2-$CH_3$-4-$SCF_2CF_3$ | 193-195 |
| *911 | i-$C_3H_7$ | H | H | 3-Cl | 4-CH=C(Cl)$CF_3$ | 196.3-208.2 |
| *943 | t-$C_4H_9$ | H | H | 6-I | 2-$CH_3$-4-$CF_2CF_3$ | 216-217 |
| *950 | n-$C_6H_{13}$ | H | H | 6-1 | 2-$CH_3$-4-$CF_2CF_3$ | 167.1-169.9 |
| *965 | i-$C_3H_7$ | H | H | 3-I | 2-i-$C_3H_7$-4-I | 132.5 |
| *978 | i-$C_3H_7$ | H | H | H | 4-$C(CF_3)_2OH$ | 87-89 |
| *982 | $CH_2CH_20CH_3$ | H | H | 6-I | 2-$CH_3$-4-$CF_2CF_3$ | 166.7-169.4 |
| *1000 | i-$C_3H_7$ | H | H | 3-$NO_2$ | 4-$SOCF_3$ | 202-205 |
| *1015 | i-$C_5H_{11}$ | H | H | 3-I | 2-$CH_3$-4-$CF_2CF_3$ | 190.0-192.5 |
| *1018 | t-$C_4H_9$ | H | H | H | 2-$CH_3$-4-$OCHF_2$ | 138-140 |
| *1066 | t-$C_4H_9$ | H | H | 3-Cl | 3-$OCF_2CF_2O$-4 | 240-241 |
| *1071 | i-$C_3H_7$ | i-$C_3H_7$ | H | H | 2-$CH_3$-4-$CF_2CF_3$ | 169.1 |
| *1100 | i-$C_3H_7$ | H | H | 3-$NO_2$ | 3-$OCF_2O$-4 | 227-229 |
| *1101 | i-$C_3H_7$ | H | H | 3-Cl | 3-$OCF_2O$-4 | 243-245 |
| *1110 | i-$C_3H_7$ | H | H | 3-Cl | 4-$SCBrF_2$ | 156-158 |
| *1143 | t-$C_4H_9$ | H | H | 3-I | 3-N=C($CF_2CF_3$)O-4 | 253-254 |
| *1146 | i-$C_3H_7$ | H | H | 3-$NO_2$ | 3-$OCHFCF_2O$-4 | 190-192 |
| *1154 | t-$C_5H_{11}$ | H | H | 3-F | 2-$CH_3$-4-$OCF_3$ | 193.6-195.8 |
| *1158 | n-$C_3H_7$ | n-$C_3H_7$ | H | 3-I | 2-$CH_3$-4-$OCF_2CHF_2$ | 116.3 |
| *1161 | i-$C_3H_7$ | H | H | 3-1 | 3-$OCF_2CHFO$-4 | 161-163 |
| *1185 | $C_2H_5$ | $C_2H_5$ | H | 3-$CF_3$ | 2-$CH_3$-4-$OCF_3$ | 192-194 |
| *1203 | i-$C_3H_7$ | H | H | 5-I | 2-$CH_3$-4-$CF_2CF_3$ | 201-203 |
| *1209 | i-$C_3H_7$ | H | H | 3-Cl | 2-$C_2H_5$-4-$OCF_3$ | 205-207 |
| 1221 | i-$C_3H_7$ | H | H | 3,4-$Cl_2$ | 2-$CH_3$-4-$OCF_3$ | 199-200 |
| 1222 | i-$C_3H_7$ | H | H | 3,4-$Cl_2$ | 2-$CH_3$-4-$CF_2CF_3$ | 208-209 |

(continued)

| No | $R^1$ | $R_2$ | $R_3$ | Xn | Ym | Physical Properties (melting point: °C |
|---|---|---|---|---|---|---|
| 1223 | $i-C_3H_7$ | H | H | $3,4-Cl_2$ | $2-CH_3-4-CF(CF_3)_2$ | 228-229 |
| 1227 | $i-C_3H_7$ | H | H | 3-Cl-4-F | $2-CH_3-4-OCF_3$ | 184-186 |
| 1228 | $i-C_3H_7$ | H | H | 3-Cl-4-F | $2-CH_3-4-CF_2CF_3$ | 178-180 |
| 1229 | $i-C_3H_7$ | H | H | 3-Cl-4-F | $2-CH_3-4-CF(CF_3)_2$ | 200-201 |
| *1235 | $C_2H_5$ | $C_2H_5$ | H | $3-OCF_3$ | $2-CH_3-4-OCF_3$ | 125-126 |
| *1247 | $i-C_3H_7$ | H | H | 5-1 | $2-CH_3-4-OCF_3$ | 205-206 |
| 1256 | $i-C_3H_7$ | H | H | 3-Cl -4-F | $2-CH_3-4-CF_2CF_3$ | 235-236 |
| 1257 | $t-C_4H_9$ | H | H | 3-Cl -4-F | $2-CH_3-4-CF_2CF_3$ | 225-226 |
| 1258 | $C_2H_5$ | $C_2H_5$ | H | 3-Cl -4-F | $2-CH_3-4-CF_2CF_3$ | 155-156 |
| 1259 | $i-C_3H_7$ | H | H | 3-Cl -4-F | $2-CH_3-4-OCF_3$ | 229-231 |
| 1260 | $t-C_4H_9$ | H | H | 3-Cl -4-F | $2-CH_3-4-OCF_3$ | 237-238 |
| 1261 | $C_2H_5$ | $C_2H_5$ | H | 3-Cl-4-F | $2-CH_3-4-OCF_3$ | 140-141 |
| 1262 | $i-C_3H_7$ | H | H | 3-Cl-4-F | $2-CH_3-4_7-CF(CF_3)_2$ | 264-265 |
| 1263 | $t-C_4H_9$ | H | H | 3-C1-4-F | $2-CH_3-4-CF(CF_3)_2$ | 253-154 |
| 1264 | $C_2H_5$ | $C_2H_5$ | H | 3-Cl-4-F | $2-CH_3-4-CF(CF_3)_2$ | 158-159 |
| 1266 | $i-C_3H_7$ | H | H | 3,4-Br2 | $2-CH_3-4-CF_2CF_3$ | 162-164 |
| 1283 | $i-C_3H_7$ | H | H | $3,4-F_2$ | $2-CH_3-4-OCF_3$ | 194-195 |
| 1284 | $t-C_4H_9$ | H | H | $3,4-F_2$ | $2-CH_3-4-OCF_3$ | 216-217 |
| 1285 | $C_2H_5$ | $C_2H_5$ | H | $3,4-F_2$ | $2-CH_3-4-OCF_3$ | 156-157 |
| *1308 | $CH_2-Ph$ | H | H | 6-Cl | $2-CH_3-4-C_2F_5$ | 175 |
| *1317 | $C(CH_3)_2CH_20H$ | H | H | 3-Cl | $2-CH_3-4-C_2F_5$ | 181-182 |
| *335 | $CH_2(3-Cl-Ph)$ | H | H | 3-Cl | $2-CH_3-4-C_2F_5$ | 168 |
| *1358 | $(CH_2)_2(3-Cl-Ph)$ | H | H | 3-Cl | $2-CH_3-4-C_2F_5$ | 117 |
| *360 | $(CH_2)_2(4-Cl-Ph)$ | H | H | 3-Cl | $2-CH_3-4-C_2F_5$ | 118 |
| *1381 | $C(CH_3)_2CH_2OH$ | H | H | H | $2-CH_3-4-C_2F_5$ | 227 |
| *1388 | $i-C_3H_7$ | H | H | 3-I | $2-OCH_3-4-C_2F5$ | 152 |
| *1404 | $CH_2(2-CH_3-Ph)$ | H | H | 3-I | $2-CH_3-4-C_2F_5$ | 100 |
| *1430 | $CH_2-Ph$ | H | H | 3-I | $2-CH_3-4-OCHF_2$ | 176 |
| *1439 | $CH_2-Ph$ | $CH_3$ | H | 6-I | $2-CH_3-4-C_2F_5$ | Paste |
| *1460 | $(R)-C^*H(CH_3)-CH_2OH$ | H | H | 3-Cl | $2-CH_3-4-C_2F_5$ | 94-95 |
| *1468 | $CH_2(2-F-Ph)$ | H | H | 3-Cl | $2-CH_3-4-C_2F_5$ | 187 |
| *1503 | $C_2H_5$ | H | H | 3-I | $2-Cl-4-C_2F_5$ | 155 |
| *508 | $t-C_4H_9$ | H | H | 3-F | $2-Cl-4-C_2F_5$ | 222 |
| *1509 | $t-C_4H_9$ | H | H | 3-F | $2-F-4-n-C_3F_7$ | 179 |
| *1542 | $C_2H_5$ | $C_2H_5$ | H | 3-Cl | $2-CH_3-Cl$ | 142 |
| *1545 | $C_2H_5$ | $C_2H_5$ | H | 3-Cl | $4-CF_3$ | 188 |
| *1546 | $C_2H_5$ | $C_2H_5$ | H | 3-F | $2-CH_3-4-Cl$ | 135 |
| *1549 | $C_2H_5$ | $C_2H_5$ | H | 3-F | $2-C_2H_5-4-C_2F_5$ | 80 |

(continued)

| No | $R^1$ | $R_2$ | $R_3$ | Xn | Ym | Physical Properties (melting point: °C |
|---|---|---|---|---|---|---|
| *1551 | $C_2H_5$ | $C_2H_5$ | H | 6-$NO_2$ | 2-$CH_3$-4-$C_2F_5$ | 145 |
| *1558 | $C_2H_5$ | $C_2H_5$ | H | 3-I | 4-$CF_3$ | 187 |
| *1576 | $CH_2$(4-$CF_3$O-Ph) | H | H | 3-Cl | 2-$CH_3$-4-$C_2F_5$ | 172 |
| *1586 | $C(CH_3)_2CH{\equiv}C$ -(2,6-$Cl_2$-Ph) | H | H | 3-Cl | 2-$CH_3$-4-$C_2F_5$ | 115-117 |
| *1605 | $C(CH_3)_2CH_2Br$ | H | H | 3-1 | 2-$CH_3$-4-$OCF_3$ | 139-141 |
| *1618 | i-$C_3H_7$ | H | H | 3-I | 2-$CH_3$-4-O-(2-Pym) | 239 |
| *1623 | i-$C_3H_7$ | H | H | 3-Cl | 2-Cl-4-$OCF_2$O-5 | 223 |
| *1624 | t-$C_4H_9$ | H | H | 3-I | 2-Cl-4-$OCF_2$O-5 | 221 |
| *1636 | i-$C_3H_7$ | H | H | 3-I | 4-N=(n-$C_3F_7$)C-O-5 | 182 |
| *700 | i-$C_3H_7$ | H | H | 3-1 | 2-$SCH_3$-4-$C_2F_5$ | 200 |
| *1737 | i-$C_3H_7$ | H | H | 3-I | 4-C($CH_3$) =$NOCH_3$ | 190 |
| *1747 | t-$C_4H_9$ | H | H | 3-1 | 2-$OCF_2$O-3-4-Cl | 235 |
| 1843 | i-$C_3H_7$ | H | H | 3-C1-4-F | 2-$CH_3$-4-$OCF_2CF_3$ | |
| 1844 | i-$C_3H_7$ | H | H | 3,4-$Cl_2$ | 2-$CH_3$-4-$OCF_2CF_3$ | |
| 1856 | t-$C_4H_9$ | H | H | 3-Cl-4-F | 2-$CH_3$-4-O-n-$C_3F_7$ | |
| 1857 | $C_2H_5$ | $C_2H_5$ | H | 3,4-$Cl_2$ | 2-$CH_3$-4-O-n-$C_3F_7$ | |
| 1900 | i-$C_3H_7$ | H | H | 3-Br -4-Cl | 2-$CH_3$-4-i-$C_3F_7$ | |
| 1901 | i-$C_3H_7$ | H | H | 3-I-4-F | 2-$CH_3$-4-i-$C_3F_7$ | |
| 1902 | i-$C_3H_7$ | H | H | 3-I-4-Cl | 2-$CH_3$-4-i-$C_3F_7$ | |
| 1903 | i-$C_3H_7$ | H | H | 3-I-4-$CF_3$ | 2-$CH_3$-4-i-$C_3F_7$ | |
| 1904 | i-$C_3H_7$ | H | H | 3-I-4-$OCH_3$ | 2-$CH_3$-4-i-$C_3F_7$ | |
| 1905 | i-$C_3H_7$ | H | H | 3-I-4-Br | 2-$CH_3$-4-i-$C_3F_7$ | |
| 1906 | i-$C_3H_7$ | H | H | 3-Cl-4-$CF_3$ | 2-$CH_3$-4-i-$C_3F_7$ | |
| 1907 | i-$C_3H_7$ | H | H | 3-$CF_3$-4-Cl | 2-$CH_3$-4-i-$C_3F_7$ | |
| 1908 | i-$C_3H_7$ | H | H | 3-$CF_3$-4-F | 2-$CH_3$-4-i-$C_3F_7$ | |
| 1919 | i-$C_3H_7$ | H | H | 3-$CF_3$-4-$OCH_3$ | 2-$CH_3$-4-i-$C_3F_7$ | |
| 1910 | i-$C_3H_7$ | H | H | 3-N=CH-CH=CH-4 | 2-$CH_3$-4-i-$C_3F_7$ | |
| 1911 | i-$C_3H_7$ | H | H | 3-$OCH_2$O-4 | 2-$CH_3$-4-i-$C_3F_7$ | |
| 1912 | i-$C_3H_7$ | H | H | 3-$OCH_2$O-4 | 2-$CH_3$-4-$C_2F_5$ | |
| 1913 | i-$C_3H_7$ | H | H | 3-$OCH_2$O-4 | 2-$CH_3$-4-$OCF_3$ | |
| 1914 | i-$C_3H_7$ | H | H | 3-$OCF_2$O-4 | 2-$CH_3$-4-i-$C_3F_7$ | |
| 1915 | i-$C_3H_7$ | H | H | 3-$OCF_2$O-4 | 2-$CH_3$-4-$C_2F_5$ | |
| 1916 | i-$C_3H_7$ | H | H | 3-$OCF_2$O-4 | 2-$CH_3$-4-$OCF_3$ | |
| 1917 | i-$C_3H_7$ | H | H | 3-$OCH_2CH_2$O-4 | 2-$CH_3$-4-i-$C_3F_7$ | |
| 1918 | i-$C_3H_7$ | H | H | 3-$OCF_2CF_2$O-4 | 2-$CH_3$-4-i-$C_3F_7$ | |
| 1919 | i-$C_3H_7$ | H | H | 3-$OCHFCF_2$O-4 | 2-$CH_3$-4-i-$C_3F_7$ | |
| 1920 | i-$C_3H_7$ | H | H | 3-$OCF_2$CHFO-4 | 2-$CH_3$-4-i-$C_3F_7$ | |
| 1921 | i-$C_3H_7$ | H | H | 3-$OCH_2CH_2$-4 | 2-$CH_3$-4-i-$C_3F_7$ | |
| 1922 | i-$C_3H_7$ | H | H | 3-$CH_2CH_2$O-4 | 2-$CH_3$-4-i-$C_3F_7$ | |

(continued)

| No | $R^1$ | $R_2$ | $R_3$ | Xn | Ym | Physical Properties (melting point: °C |
|---|---|---|---|---|---|---|
| 1923 | $i-C_3H_7$ | H | H | $3-OCF_2CF_2-4$ | $2-CH_3-4-i-C_3F_7$ | |
| 1924 | $i-C_3H_7$ | H | H | $3-CF_2CF_2O-4$ | $2-CH_3-4-i-C_3F_7$ | |
| * comparative compound | | | | | | |

The abbreviations in Table 1 stand for the following substituents:

Ph : phenyl group,
c- : alicyclic hydrocarbon group,
Pyi : pyridyl group,
Pym : pyrimidinyl group,
Fur : furyl group,
TetFur : tetrahydrofuryl group,
Thi : thienyl group,
Thz : thiazolyl group,
Naph : naphthyl group,
Oxa : oxazolyl group,
C* : asymmetric carbon atom

Agricultural and horticultural insecticides containing the phthalic acid diamide derivative of the general formula (I-2) and general formula (I-3) of the present invention as an active ingredient are suitable for controlling various insect pests such as agricultural insect pests, forest insect pests, horticultural insect pests, stored grain insect pests, sanitary insect pests, nematodes, etc., which are injurious to paddy rice, fruit trees, vegetables, other crops, flowers and ornamental plants, etc. They have a marked insecticidal effect, for example, on LEPIDOPTERA including summer fruit tortrix (Adoxophyes orana fasciata), smaller tea tortrix (Adoxophyes sp.), Manchurian fruit moth (Grapholita inopinata), oriental fruit moth (Grapholita molesta), soybean pod border (Leguminivora glycinivorella), mulberry leafroller (Olethreutes mori), tea leafroller (Caloptilia thevivora), Caloptilia sp. (Calopilia zachrysa), apple leafminer (Phyllonorycter ringoniella), pear barkminer (Spulerrina astaurota), common white (Piers rapae crucivora), tabacco budworm (Heliothis sp.), codling moth (Laspey resia pomonella), diamondback moth (Plutella xylostella), apple fruit moth (Argyresthia conjugella), peach fruit moth (Carposina niponensis), rice stem borer (Chilo suppressalis), rice leafroller (Cnaphalocrocis medinalis), tabacco moth (Ephestia elutella), mulberry pyralid (Glyphodes pyloalis), yellow rice borer (Scirpophaga incertulas), rice skipper (Parnara guttata), rice armyworm (Pseudaletia separata), pink borer (Sesamia inferens), common cutworm (Spodoptera litura), beet armyworm (Spodoptera exigua), etc.; HEMIPTERA including aster leafhopper (Macrosteles fascifrons), green rice leafhopper (Nephotettix cincticeps), brown rice planthopper (Nilaparvata lugens), whitebacked rice planthopper (Sogatella furcifera), citrus psylla (Diaphorina citri), grape whitefly (Aleurolobus taonabae), sweetpotato whitefly (Bemisia tabaci), greenhouse whitefly (Trialeurodes vaporariorum), turnip aphid (Lipaphis erysimi), green peach aphid (Myzus persicae), Indian wax scale (Ceroplastes ceriferus), cottony citrus scale (Pulvinaria aurantii), camphor scale (Pseudaonidia duplex), San Jose scale (Comstockaspis perniciosa), arrowhead scale (Unaspis yanonensis), etc.; COLEOPTERA including soybean beetle (Anomala rufocuprea), Japanese beetle (Popillia japonica), tabacco beetle (Lasioderma serricorne), powderpost beetle (Lyctus brunneus), twenty-eight-spotted ladybird (Epilachna vigintiotopunctata), adzuki bean weevile (Callosobruchus chinensis), vegetable weevil (Listroderes costirostris), maize weevil (Sitophilus zeamais), boll weevil (Anthonomus gradis gradis), rice water weevil (Lissorhoptrus oryzophilus), cucurbit leaf beetle (Aulacophora femoralis), rice leaf beetle (Oulema oryzae), striped flea beetle (Phyllotreta striolata), pine shoot beetle (Tomicus piniperda), Colorado potato beetle (Leptinotarsa decemlineata), Mexican bean beetle (Epilachna varivestis), corn rootworm (Diabrotica sp.), etc.; DIPTERA including melon fly (Dacus(Zeugodacus) cucurbitae), oriental fruit fly (Dacus(Bactrocera) dorsalis), rice leafminer (Agnomyza oryzae), onion maggot (Delia antiqua), seedcorn maggot (Delia platura), soybean pod gall midge (Asphondylia sp.), muscid fly (Musca domestica), house mosquito (Culex pipiens pipiens), etc.; and TYLENCHIDA including root-lesion nematode (Pratylenchus sp.), coffer root-lesion nematode (Pratylenchus coffeae), potato cyst nematode (Globodera rostochiensis), root-knot nematode (Meloidogyne sp.), citrus nematode (Tylenchulus semipenetrans), Aphelenchus sp. (Aphelenchus avenae), chrysanthemum foliar (Aphelenchoides ritzemabosi), etc.

The agricultural and horticultural insecticide containing the phthalic acid diamide derivative of the general

formula (I) of the present invention as an active ingredient has a marked insecticidal effect on the above-exemplified insect pests, sanitary insect pests, and/or nematodes, which are injurious to paddy field crops, upland crops, fruit trees, vegetables, other crops, flowers and ornament plants, and the like. Therefore, the desired effect of the agricultural and horticultural insecticide of the present invention can be obtained by applying the insecticide to the paddy field water, stalks and leaves of fruit trees, vegetables, other crops, flowers and ornament plants, soil, etc. at a season at which the insect pests, sanitary pests or nematodes are expected to appear, before their appearance or at the time when their appearance is confirmed.

In general, the agricultural and horticultural insecticide of the present invention is used after being prepared into conveniently usable forms according to an ordinary manner for preparation of agrochemicals.

That is, the phthalic acid diamide derivative of the general formula (I) and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in this invention may be either solid or liquid. As the solid carrier, there can be exemplified soybean flour, cereal flour, wood flour, bark flour, saw dust, powdered tobacco stalks, powdered walnut shells, bran, powdered cellulose, extraction residues of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan mono-laurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The agricultural and horticultural insecticide of the present invention is used to control a variety of insect pests in the following manner. That is, it is applied to a crop on which the insect pests are expected to appear or a site where the appearance of the insect pests is undesirable, as it is or after being properly diluted with or suspended in water or the like, in an amount effective for control of the insect pests.

The applying dosage of the agricultural and horticultural insecticide of the present invention is varied depending upon various factors such as a purpose, insect pests to be controlled, a growth state of a plant, tendency of insect pests appearance, weather, environmental conditions, a preparation form, an application method, an application site and an application time. It may be properly chosen in a range of 0.1 g to 10 kg (in terms of the active ingredient) per 10 ares depending upon purposes.

The agricultural and horticultural insecticide of the present invention may be used in admixture with other agricultural and horticultural disease or pest controllers in order to expand both spectrum of controllable diseases and insect pest species and the period of time when effective applications are possible or to reduce the dosage.
Typical examples of the present invention are described below, but they should not be construed as limiting the scope of the invention.

EXAMPLES

Example 1 (Comparative)

(1-1) Production of N-(4-trifluoromethoxyphenyl)-3-nitrophthalimide

In 50 ml of acetic acid were dissolved 5.97 g of 3-nitrophthalic anhydride and 5.31 g of 4-trifluoromethoxyaniline, and the reaction was carried out with heating under reflux for 3 hours. After completion of the reaction, the solvent was distilled off under reduced pressure and the resulting residue was washed with an ether-hexane mixed solvent to obtain 10.2 g of the desired compound.
Physical property: m.p. 149 - 150°C.
Yield: 97%.

(1-2) Production of 3-amino-N-(4-trifluoromethoxyphenyl)phthalimide

In a pressure vessel were placed 10.0 g of N-(4-trifluoromethoxyphenyl)-3-nitrophthalimide, 100 ml of acetic acid and 0.5 g of 5% palladium carbon, and catalytic reduction with hydrogen was carried out at a hydrogen pressure of 5 $kg/cm^2$. After completion of the reaction, the catalyst was filtered off and the filtrate was concentrated under reduced pressure. The resulting residue was washed with an ether-hexane mixed solvent to obtain 9.0 g of the desired compound.
Physical property: m.p. 161 - 162°C.
Yield: 98%.

(1-3) Production of 3-bromo-N-(4-trifluoromethoxyphenyl)phthalimide

In 20 ml of acetic acid was dissolved 1.6 g of 3-amino-N-(4-trifluoromethoxyphenyl)phthalimide, and a solution of 0.35 g of sodium nitrite in 5 ml of concentrated sulfuric acid was added dropwise while maintaining the temperature at 15°C or lower. The resulting mixture was stirred at 15°C or lower for another 20 minutes to obtain a diazonium salt. The diazonium salt was slowly added to a mixture of a solution of 0.86 g of cuprous bromide in 50 ml of hydrobromic acid and 10 ml of toluene which was maintained at 80°C. The resulting mixture was stirred until foaming ceased. After completion of the reaction, the organic layer was washed with an aqueous sodium thiosulfate solution and an aqueous sodium chloride solution, dried over anhydrous magnesium sulfate, and then distilled under reduced pressure to remove the solvent, and the resulting residue was purified by a silica gel chromatography to obtain 1.3 g of the desired compound.
Physical property: m.p. 117 - 118°C.
Yield: 67%.

(1-4) Production of 3-bromo-$N^1$-(4-trifluoromethoxyphenyl)-$N^2$-isopropylphthalic acid diamide (compound No. 262) and 6-bromo-$N^1$-(4-trifluoromethoxyphenyl)-$N^2$-isopropylphthalic acid diamide (compound No. 302)

From 1.3 g of 3-bromo-N-(4-trifluoromethoxyphenyl)phthalimide, 0.5 g of the desired compound (compound No. 262) and 0.7 g of the other desired compound (compound No. 302) were obtained in the same manner as in Example 1-2.
Compound No. 262:

Physical property: m.p. 208 - 210°C.
Yield: 33%.

Compound No. 302:

Physical property: m.p. 210 - 212°C.
Yield: 47%.

Example 2

(2-1) Production of N-isopropyl-3,4-dichlorophthalamic acid

In 30 ml of tetrahydrofuran was dissolved 2.32 g of N-isopropyl-3,4-dichlorobenzamide, and 21 ml of s-BuLi (0.96 M/L) was slowly added while maintaining the temperature at -70°C. The resulting mixture was stirred at -70°C for 30 minutes, after which the cooling bath was removed. An excess of carbon dioxide was introduced into the reaction solution, and the thus treated solution was stirred at room temperature for 30 minutes to carry out the reaction.

After completion of the reaction, the reaction solution was poured into water and acidified with diluted hydrochloric acid, and the desired compound was extracted with ethyl acetate. The extracted solution was dried over anhydrous magnesium sulfate and distilled under reduced pressure to remove the solvent, and the crystals thus obtained were washed with an ether-hexane mixed solvent to obtain 2.4 g of the desired compound.
Physical property: m.p. 155 - 156°C.
Yield: 86.9%.

(2-2) Production of N-isopropyl-3,4-dichlorophthalic acid isoimide

In 10 ml of toluene was dissolved 0.41 g of N-isopropyl-3,4-dichlorophthalamic acid, followed by adding thereto 0.42 g of trifluoroacetic anhydride, and the reaction was carried out with stirring at room temperature for 30 minutes. After completion of the reaction, the solvent was distilled off under reduced pressure to obtain 0.39 g of the desired compound as a crude product. The obtained desired compound was used in the subsequent reaction without purification.

(2-3) Production of 3,4-dichloro-$N^1$-(4-pentafluoroethyl-2-methylphenyl)-$N^2$-isopropylphthalic acid diamide (compound No. 1222)

In 10 ml of acetonitrile was dissolved 0.39 g of N-isopropyl-3,4-dichlorophthalic acid isoimide, followed by adding thereto 0.34 g of 4-pentafluoroethyl-2-methylaniline, and the reaction was carried out with stirring for 2 hours. After completion of the reaction, the reaction solution was maintained at 0°C for 10 minutes and the crystals precipitated were collected by filtration and washed with hexane to obtain 0.61 g of the desired compound.
Physical property: m.p. 208 - 209°C.
Yield: 84.1%.

Typical preparation examples and test examples of the present invention are described below but they should not be construed as limiting the scope of the invention.

In the preparation examples, parts are all by weight.

Formulation Example 1

| | |
|---|---|
| Each compound listed in Table 1 | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

Formulation Example 2

| | |
|---|---|
| Each compound listed in Table 1 | 3 parts |
| Clay powder | 82 parts |
| Diatomaceous earth powder | 15 parts |

A dust was prepared by mixing uniformly and grinding the above ingredients.

Formulation Example 3

| | |
|---|---|
| Each compound listed in Table 1 | 5 parts |
| Mixed powder of bentonite and clay | 90 parts |
| Calcium lignin sulfonate | 5 parts |

Granules were prepared by mixing the above ingredients uniformly, and kneading the resulting mixture together

with a suitable amount of water, followed by granulation and drying.

Formulation Example 4

| | |
|---|---|
| Each compound listed in Table 1 | 20 parts |
| Mixture of kaolin and synthetic high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1

Insecticidal effect on diamondback moth (Plutella xylostella)

Adult diamondback moths were released and allowed to oviposit on a Chinese cabbage seedling. Two days after the release, the seedling having eggs deposited thereon was immersed for about 30 seconds in a liquid chemical prepared by diluting a preparation containing each compound listed in Table 1 as an active ingredient to adjust the concentration to 500 ppm. After air-drying, it was allowed to stand in a room thermostated at 25°C. Six days after the immersion, the hatched insects were counted. The mortality was calculated according to the following equation and the insecticidal effect was judged according to the criterion shown below. The test was carried out with triplicate groups of 10 insects.

$$\text{Corrected mortality (\%)} = \frac{\begin{bmatrix}\text{Number of}\\ \text{hatched insects}\\ \text{in untreated group}\end{bmatrix} - \begin{bmatrix}\text{Number of}\\ \text{hatched insects}\\ \text{in treated group}\end{bmatrix}}{\begin{bmatrix}\text{Number of}\\ \text{hatched insects}\\ \text{in untreated group}\end{bmatrix}} \times 100$$

Criterion:

| Effect | Mortality(%) |
|---|---|
| A | 100 |
| B | 99 - 90 |
| C | 89 - 80 |
| D | 79 - 50 |

The results obtained are shown in Table 4.

Test Example 2

Insecticidal effect on common cutworm (<u>Spodoptera Litura)</u>

A piece of cabbage leaf (cultivar; Shikidori) was immersed for about 30 seconds in a liquid chemical prepared by diluting a preparation containing each compound listed in Table 1 as an active ingredient to adjust the concentration to 500 ppm. After air-drying, it was placed in a plastic Petri dish with a diameter of 9 cm and inoculated with second-instar larvae of common cutworm, after which the dish was closed and then allowed to stand in a room thermostated at 25°C. Eight days after the inoculation, the dead and alive were counted. The mortality was calculated according to the following equation and the insecticidal effect was judged according to the criterion shown in Test Example 1. The test was carried out with triplicate groups of 10 insects.

$$\text{Corrected mortality (\%)} = \frac{\left[\begin{array}{l}\text{Number of}\\ \text{alive larvae in}\\ \text{untreated group}\end{array}\right] - \left[\begin{array}{l}\text{Number of}\\ \text{alive larvae in}\\ \text{treated group}\end{array}\right]}{\left[\begin{array}{l}\text{Number of}\\ \text{alive larvae in}\\ \text{untreated group}\end{array}\right]} \times 100$$

The results obtained are shown in Table 4.

Test Example 3

Insecticidal effect on rice leafroller (Cnaphalocrocis medinalis)

The lamina of a rice plant at the 6 to 8 leaf stage was immersed for about 30 seconds in a liquid chemical prepared by diluting a preparation containing each compound listed in Table 1 as an active ingredient to adjust the concentration to 500 ppm. After air-drying, the lamina was placed in a plastic Petri dish with a diameter of 9 cm whose bottom had been covered with a wetted filter paper. The lamina was inoculated with third-instar larvae of rice leafroller, after which the dish was allowed to stand in a room thermostated at 25°C and having a humidity of 70%. Four days after the inoculation, the dead and alive were counted and the insecticidal effect was judged according to the criterion shown in Test Example 1. The test was carried out with triplicate groups of 10 insects.

The results obtained are shown in Table 2;

Table 2

| No | Test Example 1 | Test Example 2 | Test Example 3 |
|---|---|---|---|
| *1 | D | D | A |
| *2 | A | C | |
| *3 | C | A | |
| *4 | A | | D |
| *10 | A | D | D |
| *11 | A | C | C |
| *12 | A | D | |
| *13 | D | | D |
| *17 | A | | D |
| *18 | D | | A |
| *22 | A | D | |
| *23 | A | | D |
| *24 | A | | D |
| *26 | A | | D |
| *27 | A | A | C |
| *34 | A | C | |
| *42 | A | D | A |
| *43 | B | D | |
| *47 | A | D | A |
| *71 | | | D |
| *76 | C | | B |
| *77 | A | C | A |
| *79 | A | A | D |
| *98 | A | | C |
| *102 | A | D | A |
| *103 | A | C | A |

(continued)

| No | Test Example 1 | Test Example 2 | Test Example 3 |
|---|---|---|---|
| *109 | A | A | C |
| *111 | A | C | B |
| *115 | A | C | A |
| *116 | A | D | A |
| *120 | A | D | A |
| *128 | A | D | A |
| *147 | A | C | |
| *153 | A | | D |
| *161 | A | D | A |
| *165 | A | B | C |
| *169 | A | D | |
| *170 | A | D | B |
| *171 | A | | D |
| *172 | A | A | D |
| *173 | A | D | D |
| *176 | A | D | A |
| *186 | D | | |
| *187 | A | | D |
| *188 | D | | D |
| *193 | A | D | |
| *196 | A | | D |
| *198 | A | C | A |
| *210 | A | | D |
| *212 | A | D | A |
| *215 | A | D | |
| *218 | A | | C |
| *219 | A | D | A |
| *229 | A | D | A |
| *230 | A | C | A |
| *244 | A | C | |
| *245 | A | D | |
| *246 | A | B | B |
| *248 | A | C | |
| *249 | A | D | A |
| *250 | A | | D |
| *253 | A | A | C |
| *259 | A | | D |
| *261 | A | A | D |
| *262 | A | A | D |
| *264 | - | D | A |
| *273 | A | D | D |
| *275 | A | D | A |
| *285 | A | D | A |
| *295 | D | | |
| *301 | A | A | D |
| *302 | A | | D |
| *303 | A | | D |
| *307 | A | | D |
| *311 | A | | D |
| *319 | A | B | B |

(continued)

| No | Test Example 1 | Test Example 2 | Test Example 3 |
|---|---|---|---|
| *320 | A | | D |
| *323 | A | C | A |
| *333 | A | | D |
| *334 | A | C | C |
| *336 | A | | D |
| 338 | A | B | A |
| 339 | A | B | A |
| *346 | A | C | A |
| *347 | A | B | C |
| *355 | A | C | A |
| *360 | A | D | A |
| *364 | A | A | D |
| *378 | A | D | A |
| *384 | A | | C |
| *400 | A | D | A |
| *412 | A | | C |
| *413 | A | | C |
| *420 | A | | D |
| *427 | A | | D |
| *429 | A | D | |
| *430 | A | D | D |
| *437 | A | C | A |
| *440 | A | C | B |
| *443 | A | | D |
| *447 | A | B | C |
| *450 | A | | C |
| *453 | A | D | A |
| *469 | A | A | D |
| *470 | A | C | C |
| *475 | A | | D |
| *477 | A | | C |
| *515 | A | | C |
| *522 | A | D | A |
| *529 | A | D | A |
| *530 | A | | D |
| *547 | A | A | D |
| *549 | A | A | D |
| *550 | A | C | A |
| *553 | A | C | A |
| *557 | A | C | B |
| *561 | A | C | A |
| *567 | A | D | D |
| *568 | A | C | A |
| *571 | A | C | |
| *576 | A | | C |
| *584 | A | D | |
| *586 | A | | D |
| *591 | A | D | |
| *596 | D | | D |
| *599 | A | D | A |

(continued)

| No | Test Example 1 | Test Example 2 | Test Example 3 |
|---|---|---|---|
| *603 | B | | C |
| *604 | A | | D |
| *605 | | | C |
| *606 | A | D | A |
| *610 | A | A | C |
| *612 | A | | D |
| *616 | A | | D |
| *625 | A | D | D |
| *633 | A | D | |
| *634 | A | | D |
| *635 | A | D | |
| *636 | A | D | A |
| *641 | D | D | |
| *646 | A | D | |
| *648 | A | D | A |
| *649 | A | | C |
| *654 | | D | |
| *657 | D | | |
| *661 | B | | D |
| *663 | A | A | D |
| *666 | A | | D |
| *669 | A | D | A |
| *670 | A | | D |
| *671 | A | | D |
| *673 | A | D | D |
| *674 | A | D | A |
| *676 | A | C | A |
| *680 | A | | D |
| *687 | A | D | D |
| *699 | A | D | A |
| *691 | A | D | C |
| *692 | A | D | |
| *699 | A | A | D |
| *705 | A | D | A |
| *708 | D | | |
| *710 | A | C | A |
| *711 | A | C | A |
| *713 | A | B | D |
| *719 | A | D | |
| *723 | D | | D |
| *735 | | | D |
| *738 | D | | |
| *743 | D | | |
| *744 | C | | |
| *745 | D | | |
| *753 | A | A | D |
| *759 | | | D |
| *767 | A | C | A |
| *769 | A | | D |
| *771 | A | | C |

(continued)

| No | Test Example 1 | Test Example 2 | Test Example 3 |
|---|---|---|---|
| *776 | B | | D |
| *777 | A | | D |
| *788 | C | | C |
| *797 | A | | C |
| *800 | | | C |
| *801 | A | A | D |
| *802 | D | | |
| 819 | A | B | A |
| 821 | A | | A |
| *822 | D | | D |
| *830 | C | | |
| *831 | D | D | |
| *833 | A | | D |
| *838 | A | C | A |
| *839 | A | | C |
| *840 | A | | D |
| *841 | A | D | |
| *842 | A | A | D |
| *847 | | D | |
| *851 | A | D | A |
| *852 | A | | D |
| *856 | A | | D |
| *858 | C | A | |
| *859 | D | | |
| *862 | A | D | D |
| *866 | D | | |
| *867 | A | | C |
| *869 | A | D | |
| *872 | A | | C |
| *874 | A | C | A |
| *878 | C | | |
| *880 | | D | |
| *881 | A | D | |
| *888 | D | | |
| *901 | A | D | A |
| *906 | A | D | A |
| *908 | A | D | A |
| *911 | A | | D |
| *943 | A | C | A |
| *950 | | | C |
| *965 | A | C | |
| *978 | A | C | A |
| *982 | A | | C |
| *1000 | A | D | A |
| *1015 | A | D | A |
| *1018 | A | D | A |
| *1066 | A | C | A |
| *1071 | A | C | A |
| *1100 | A | C | A |
| *1101 | A | C | A |

(continued)

| No | Test Example 1 | Test Example 2 | Test Example 3 |
|---|---|---|---|
| *1110 | A | C | A |
| *1143 | A | C | A |
| *1146 | A | C | A |
| +1154 | A | C | A |
| *1158 | A | D | A |
| *1161 | A | D | A |
| *1185 | A | C | - |
| *1203 | A | D | A |
| *1209 | A | D | A |
| 1221 | A | A | A |
| 1222 | A | A | A |
| 1223 | A | A | A |
| 1227 | A | A | A |
| 1228 | A | A | A |
| 1229 | A | A | A |
| *1235 | A | D | - |
| *1247 | C | | |
| 1256 | A | A | - |
| 1257 | A | A | - |
| 1258 | A | A | - |
| 1259 | A | | - |
| 1260 | A | | - |
| 1261 | A | A | - |
| 1262 | A | A | A |
| 1263 | A | A | A |
| 1264 | A | A | A |
| 1266 | A | | A |
| 1283 | A | - | - |
| 1284 | A | - | A |
| 1285 | A | - | A |
| *1308 | | | C |
| *1317 | A | C | A |
| *1335 | A | C | A |
| *1358 | A | | C |
| *1360 | A | | C |
| *1381 | A | | C |
| *1388 | A | D | A |
| *1404 | A | C | A |
| *1430 | A | D | A |
| *1439 | A | | C |
| *1460 | A | C | A |
| *1468 | A | C | A |
| *1503 | A | C | A |
| *1508 | A | C | A |
| *1509 | A | C | A |
| *1542 | A | D | A |
| *1545 | A | D | A |
| *1546 | A | C | A |
| *1549 | A | D | A |
| *1551 | A | D | A |

(continued)

| No | Test Example 1 | Test Example 2 | Test Example 3 |
|---|---|---|---|
| *1558 | A | C | A |
| *1576 | A | D | A |
| *1586 | A | C | A |
| *1605 | A | C | - |
| *1614 | A | C | A |
| *1618 | A | C | A |
| *1623 | A | C | A |
| *1624 | A | D | A |
| *1636 | A | D | A |
| *1700 | A | D | - |
| *1737 | A | C | A |
| *1747 | A | C | A |
| * comparative compound | | | |

In Table 4, "-" means that test is not conducted.

## Claims

1. A phthalic acid diamide derivative, represented by the general formula (I-2)

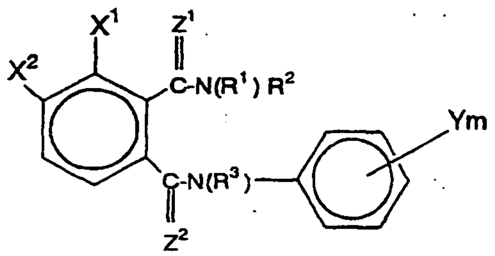

(I-2)

{wherein

$R^1$, $R^2$ and $R^3$ may be the same or different, and are each a hydrogen atom; a $C_3$-$C_6$ cycloalkyl group; a halo-$C_3$-$C_6$ cycloalkyl group or a group of the formula -$A^1$-$Q_l$ (wherein $A^1$ is a $C_1$-$C_8$ alkylene group; a $C_3$-$C_6$ alkenylene group or a $C_3$-$C_6$ alkynylene group;

Q is a hydrogen atom; a halogen atom; a cyano group; a nitro group; a halo-$C_1$-$C_6$ alkyl group; a $C_3$-$C_6$ cycloalkyl group; a halo-$C_3$-$C_6$ cycloalkyl group: a $C_1$-$C_6$ alkoxycarbonyl group; a di-$C_1$-$C_6$ alkoxyphosphoryl group which may be the same or different; a di-$C_1$-$C_6$ alkoxythiophosphoryl group which may be the same or different; a diphenylphosphino group; a diphenylphosphono group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group; a heterocyclic group (wherein the heterocyclic group is defined as below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo- $C_1$-$C_6$ alkyl-sulfonyl group; or a group of the formula -$Z^3$ -$R^5$

(wherein $Z^3$ is -O-; -S-; -SO-; -$SO_2$- or a group of the formula -N($R^6$)- (wherein $R^6$ is a hydrogen atom; a $C_1$-$C_6$ alkylcarbonyl group; a halo-$C_1$-$C_6$ alkylcarbonyl group; a $C_1$-$C_6$ alkoxycarbonyl group; a phenylcarbonyl group;

a substituted phenylcarbonyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenyl $C_1$-$C_4$ alkoxycarbonyl group or a substituted phenyl $C_1$-$C_4$ alkoxycarbonyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group); and

$R^5$ is a hydrogen atom; a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$ alkynyl group; a halo-$C_3$-$C_6$ alkynyl group; a $C_3$-$C_6$ cycloalkyl group; a halo-$C_3$-$C_6$ cycloalkyl group; a $C_1$-$C_6$ alkylcarbonyl group; a halo- $C_1$-$C_6$ alkylcarbonyl group; a $C_1$-$C_6$ alkoxycarbonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenyl $C_1$-$C_4$ alkyl group; a substituted phenyl $C_1$-$C_4$ alkyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); or a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group; a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkyl-sulfonyl group); and

1 is an integer of 1 to 4); further,

$R^1$ and $R^2$ may form a 4 to 7 membered ring by combining to each other, in which the ring may contain the same or different 1 to 3 heteroatoms selected from the group consisting of oxygen atom, sulfur atom and nitrogen atom;

$X^1$ and $X^2$ may be the same or different and are each a halogen atom; a cyano group; a $C_1$-$C_6$ alkyl group; a halo-$C_1$-$C_6$ alkyl group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group and a halo-$C_1$-$C_6$ alkylsulfonyl group; further,

$X^1$ and $X^2$ may form a condensed ring with the phenyl ring of phthalic acid diamide derivative (which means naphthalene, tetrahydronaphthalene, indene, indane, quinoline, quinazoline, chroman, isochroman, indole, indoline, benzodioxane, benzodioxole, benzofuran, dihydrobenzofuran, benzothiophene, dihydrobenzothiophene, benzoxazole, benzothiazole, benzimidazole, or indazole) by combining to each other, and said condensed ring may have at least one substituent, which may be the same or different and is selected from the group consisting of a halogen atom; a $C_1$-$C_6$ alkyl group; a halo-$C_1$-$C_6$ alkylgroup; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkylsulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group a $C_1$-$C_6$ alkylsulfonyl group and a halo-$C_1$-$C_6$ alkylsulfonyl group; a heterocyclic group (which is the same as defined below); and a substituted heterocyclic group (wherein the heterocyclic group is the same as defined above) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group;

Y is the same or different, and are each a hydrogen atom; a halogen atom; a cyano group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of -$A^2$-$R^7$ (wherein $A^2$ is -O-; -S-; -SO-; - $SO_2$-; -C(=O)-; -C(=$NOR^8$)- (wherein $R^8$ is a hydrogen atom; a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$ alkynyl group; a $C_3$-$C_6$ cycloalkyl group; a phenyl-$C_1$-$C_4$ alkyl group; or a substituted phenyl-$C_1$-$C_4$ alkyl group having at least one substituent, in the phenyl ring, which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group); a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group or a halo-$C_3$-$C_6$ alkynylene group;

(1) when $A^2$ is -O-; -S-; -SO- or -$SO_2$-, then $R^7$ is a halo-$C_3$-$C_6$ cycloalkyl group; a halo-$C_3$-$C_6$ cycloalkenyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of the formula - $A^3$-$R^9$
(wherein $A^3$ is a $C_1$-$C_6$ alkylene group; a halo-$C_1$-$C_6$ alkylene group; a $C_3$-$C_6$ alkenylene group; a halo-$C_3$-$C_6$ alkenylene group; a $C_3$-$C_9$ alkynylene group or a halo-$C_3$-$C_6$ alkynylene group;
$R^9$ is a hydrogen atom; a halogen atom; a $C_3$-$C_6$ cycloalkyl group; a halo-$C_3$-$C_6$ cycloalkyl group; a $C_1$-$C_6$ alkoxycarbonyl group; a phenyl group; a substituted phenyl group having at least one substituent which may be the same or different and is selected from the group consisting of a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkylsulfonyl group or a group of the formula -$A^4$-$R^{10}$
(wherein $A^4$ is -O-; -S-; -SO-; -$SO_2$- or -C(=O)-, and
$R^{10}$ is a $C_1$-$C_6$ alkyl group; a halo- $C_1$-$C_6$ alkyl group; a $C_3$-$C_6$ alkenyl group; a halo-$C_3$-$C_6$ alkenyl group; a $C_3$-$C_6$ cycloalkyl group; a halo-$C_3$-$C_6$ cycloalkyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); or a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group));
(2) when $A^2$ is -C(=O)- or a group of the formula -C(=$NOR^8$)- (wherein $R^8$ is the same as defined the above), then $R^7$ is a $C_1$-$C_6$ alkyl group; a halo $C_1$-$C_6$ alkyl group; a $C_2$-$C_6$ alkenyl group; a halo-$C_2$-$C_6$ alkenyl group; a $C_3$-$C_6$ cycloalkyl group; a halo-$C_3$-$C_6$ cycloalkyl group; a $C_1$-$C_6$ alkoxy group; a $C_1$-$C_6$ alkylthio group; a mono-$C_1$-$C_6$ alkylamino group; a di-$C_1$-$C_6$ alkylamino group which may be the same or different; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenylamino group; a substituted phenylamino group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$

alkylsulfonyl group, and a halo-$C_1$-$C_6$ alkylsulfonyl group; a heterocyclic group (which is the same as defined the below); or a substituted heterocyclic group (wherein the heterocyclic group is the same as defined the below) having at least one substituent which may be the same or different and is selected from the group consisting of a halogen atom, a $C_1$-$C_6$ alkyl group, a halo- $C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo- $C_1$-$C_6$ alkylsulfonyl group;

(3) when $A^2$ is a $C_1$-$C_6$ alkylene group; a halo-$C_1$-$C_6$ alkylene group; $C_2$-$C_6$ alkenylene group; a halo-$C_2$-$C_6$ alkenylene group; a $C_2$-$C_6$ alkynylene group or a halo-$C_3$-$C_6$ alkynylene group; then $R^7$ is a hydrogen atom; a halogen atom; a $C_3$-$C_6$ cycloalkyl group; a halo-$C_3$-$C_6$ cycloalkyl group; a $C_1$-$C_6$ alkoxycarbonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); a substituted heterocyclic group (wherein the heterocyclic group is the same as defined below) having at least one substituent which may be the same or different and is selected from the group consisting of a halogen atom; a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of the formula $-A^5-R^{12}$ (wherein $A^5$ is -O-; -S-; -SO- or $-SO_2-$; and

$R^{12}$ is a $C_3$-$C_6$ cycloalkyl group; a halo-$C_3$-$C_6$ cycloalkyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; or a group of the formula $-A^6-R^{14}$.

(wherein $A^6$ is a $C_1$-$C_6$ alkylene group; halo-$C_1$-$C_6$ alkylene group; a $C_2$-$C_6$ alkenylene group; a halo-$C_2$-$C_6$ alkenylene group; a $C_2$-$C_6$ alkynylene group; or a halo-$C_3$-$C_6$ alkynylene group; and

$R^{14}$ is a hydrogen atom; a halogen atom; a $C_3$-$C_6$ cycloalkyl group; a halo-$C_{3-}C_6$ cycloalkyl group; a $C_1$-$C_6$ alkoxy group; a halo-$C_1$-$C_6$ alkoxy group; a $C_1$-$C_6$ alkylthio group; a halo- $C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkyl-sulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenoxy group; a substituted phenoxy group having at least one substituent which may be the same or different and is selected from the group: consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a phenylthio group; a substituted phenylthio group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined the below); or a substituted heterocyclic group (wherein the heterocyclic ring is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group))); and

m is an integer of 1 to 5;

further, Y may form a condensed ring (which is the same as defined above) by combining together with the

adjacent carbon atoms in the phenyl ring, said condensed ring may have at least one substituent, which is the same or different and is selected from the group consisting of a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a $C_1$-$C_6$ alkylsulfinyl group; a halo-$C_1$-$C_6$ alkylsulfinyl group; a $C_1$-$C_6$ alkylsulfonyl group; a halo-$C_1$-$C_6$ alkylsulfonyl group; a phenyl group; a substituted phenyl group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group; a heterocyclic group (which is the same as defined below); and a substituted heterocyclic group (wherein the heterocyclic group is the same as defined below) having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkylsulfonyl group and a halo-$C_1$-$C_6$ alkylsulfonyl group;
$Z^1$ and $Z^2$ are each an oxygen atom or a sulfur atom; and

said heterocyclic group is selected from the group consisting of a pyridyl group, pyridine-N-oxide group, pyrimidinyl group, furyl group, tetrahydrofuryl group, thienyl group, tetrahydrothienyl group, tetrahydropyranyl group, tetrahydrothiopyranyl group, oxazolyl group, isoxazolyl group, oxadiazolyl group, thiazolyl group, isothiazolyl group, thiadiazolyl group, imidazolyl group, triazolyl group and pyrazolyl group}.

**2.** A phthalic acid diamide derivative according to claim 1, represented by the general formula (I-3),

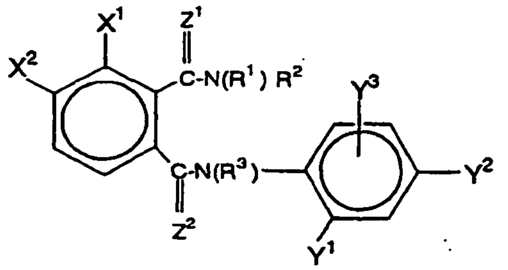

(I-3)

{wherein $R^1$, $R^2$ and $R^3$, l, $X^1$ and $X^2$ are defined as in claim 1 with respect to formula (I-2);
$Y^1$ and $Y^3$ may be the same or different, and are each a hydrogen atom; a halogen atom; a $C_1$-$C_6$ alkyl group; a halo-$C_1$-$C_6$ alkyl group; a $C_1$-$C_6$ alkoxy group; a halo-$C_1$-$C_6$ alkoxy group; a $C_1$-$C_6$ alkylthio group; a halo-$C_1$-$C_6$ alkylthio group; a phenoxy group; a substituted phenoxy group having at least one substituent which may be the same or different and is selected from the group consisting of a halogen atom, a $C_1$-$C_6$ alkyl group, a halo-$C_1$-$C_6$ alkyl group, a $C_1$-$C_6$ alkoxy group, a halo-$C_1$-$C_6$ alkoxy group, a $C_1$-$C_6$ alkylthio group, a halo-$C_1$-$C_6$ alkylthio group, a $C_1$-$C_6$ alkylsulfinyl group, a halo-$C_1$-$C_6$ alkylsulfinyl group, a $C_1$-$C_6$ alkyl-sulfonyl group and a halo-$C_1$-$C_6$ alkylsulfonyl group; a pyridyloxy group; or a substituted pyridyloxy group having at least one substituent which may be the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; $Y^2$ is a hydrogen atom; a halogen atom or a group of the formula -$A^2$-$R^7$ (wherein $A^2$ -O-; -S-; -SO-; -$SO_2$-; a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group; or a halo-$C_3$-$C_6$ alkynylene group; and

(1) when $A^2$ is -O-; -S-; -SO- or -$SO_2$-, then $R^7$ is a halo-$C_3$-$C_6$ cycloalkyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$alkylthio group, a $C_1$-$C_6$ alkylsulfinyl group and a halo-$C_1$-$C_6$ alkylsulfonyl group; a substituted pyridyloxy group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^3$-$R^9$

(wherein $A^3$ is a halo-$C_1$-$C_6$ alkylene group or a halo-$C_3$-$C_6$ alkenylene group and;
$R^9$ is a hydrogen atom; a halogen atom; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^4$-$R^{10}$
(wherein $A^4$ is -O-; -S-; -SO- or -$SO_2$-;
$R^{10}$ is a $C_1$-$C_6$ alkyl group; a halo-$C_1$-$C_6$ alkyl group; a $C_3$-$C_6$ alkenyl group; a halo-$C_3$-$C_6$ alkenyl group; a $C_3$-$C_6$ cycloalkyl group; a halo-$C_3$-$C_6$ cycloalkyl group; a phenyl group; or a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group));
(2) when $A^2$ is a $C_1$-$C_6$ alkylene group; a halo-$C_1$-$C_6$ alkylene group; a $C_2$-$C_6$ alkenylene group; a halo-$C_2$-$C_6$ alkenylene group; a $C_2$-$C_6$ alkynylene group; a halo-$C_3$-$C_6$ alkynylene group; then $R^7$ is a hydrogen atom; a halogen atom; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^5$-$R^{12}$
(wherein $A^5$ is -O-; -S-; -SO- or -$SO_2$-; and
$R^{12}$ is a $C_3$-$C_6$ cycloalkyl group; a halo-$C_3$-$C_6$ cycloalkyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; or a group of the formula -$A^6$-$R^{14}$
(wherein $A^6$ is a $C_1$-$C_6$ alkylene group; a halo-$C_1$-$C_6$ alkylene group; a $C_2$-$C_6$ alkenylene group; a halo-$C_2$-$C_6$ alkenylene group; and
$R^{14}$ is a hydrogen atom; a halogen atom; a halo-$C_3$-$C_6$ cycloalkyl group; a halo-$C_1$-$C_6$ alkoxy group; a halo-$C_1$-$C_6$ alkylthio group; a halo-$C_1$-$C_6$ alkylsulfinyl group; a halo-$C_1$-$C_6$ alkylsulfonyl group; a phenyl group; a substituted phenyl group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; a phenoxy group; a substituted phenoxy group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group; a phenylthio group; or a substituted phenylthio group having at least one substituent, which is the same or different and is selected from the group consisting of a halogen atom, a halo-$C_1$-$C_6$ alkyl group, a halo-$C_1$-$C_6$ alkoxy group, a halo-$C_1$-$C_6$ alkylthio group, a halo-$C_1$-$C_6$ alkylsulfinyl group and a halo-$C_1$-$C_6$ alkylsulfonyl group));
and further,

$Z^1$ and $Z^2$ are defined as in claim 1 with respect to formula (I-2).

**3.** Use of the phthalic acid diamide derivative according to claim 1 or 2 as agricultural and horticultural insecticide.

**4.** A method for controlling undesirable insect pests for a useful crop, **characterized by** treating an objective crop with an effective amount of the phthalic acid diamide derivative according to claim 1 or 2.

**5.** An agricultural and horticultural insecticide, comprising the phthalic acid diamide derivative according to claim 1 or 2 as an active ingredient.

**Patentansprüche**

**1.** Phthalsäurediamidderivat, dargestellt durch die allgemeine Formel (1-2)

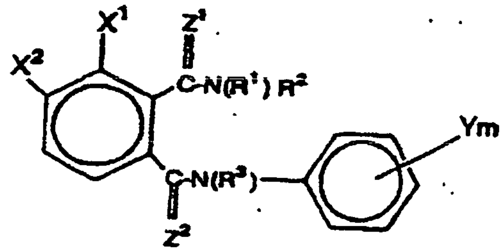

(I-2)

worin

$R^1$, $R^2$ und $R^3$ gleich oder verschieden sein können und jedes ein Wasserstoffatom; eine $C_3$-$C_6$ Cycloalkylgruppe; eine Halogen-$C_3$-$C_6$ Cycloalkylgruppe oder eine Gruppe der Formel —$A^1$-$Q_l$ (worin $A^1$ eine $C_1$-$C_8$ Alkylengruppe; eine $C_3$-$C_6$ Alkenylengruppe oder $C_3$-$C_6$ Alkinylengruppe ist;

Q ein Wasserstoffatom; ein Halogenatom; eine Cyanogruppe; eine Nitrogruppe; eine Halogen-$C_1$-$C_6$ Alkylgruppe; eine $C_3$-$C_6$ Cycloalkylgruppe; eine Halogen-$C_3$-$C_6$ Cycloalkylgruppe; eine $C_1$-$C_6$ Alkoxycarbonylgruppe; eine Di-$C_1$-$C_6$ Alkoxyphosphorylgruppe, welche gleich oder verschieden sein kann; eine Di-$C_1$-$C_6$ Alkoxythiophosphorylgruppe, welche gleich oder verschieden sein kann; eine Diphenylphosphinogruppe; eine Diphenylphosphonogruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclische Gruppe (worin die heterocyclische Gruppe wie unten definiert ist); eine substituierte heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe;

oder einer Gruppe der Formel -$Z^3$-$R^5$

(worin $Z^3$ -O-, -S-, -SO-, -$SO_2$- oder eine Gruppe der Formel -N($R^6$)- ist (worin $R^6$ ein Wasserstoffatom ist; eine $C_1$-$C_6$ Alkylcarbonylgruppe; eine Halogen-$C_1$-$C_6$ Alkylcarbonylgruppe; eine $C_1$-$C_6$ Alkoxycarbonylgruppe; eine Phenylcarbonylgruppe; eine substituierte Phenylcarbonylgruppe mit mindestens einen Substituenten, welcher gleich oder verschieden sein kann und ausgewählt ist aus der Gruppe bestehend aus einem Halogenatom, einer $C_1$-$C_6$-Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine Phenyl-$C_1$-$C_4$ Alkoxycarbonylgruppe oder eine substituierte Phenyl-$C_1$-$C_4$ Alkoxycarbonylgruppe mit mindestens einem Substituenten im Phenylring, welcher gleich oder verschieden sein kann und ausgewählt ist aus der Gruppe bestehend aus einem Halogenatom; einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe); und

$R^5$ ist ein Wasserstoffatom; eine $C_1$-$C_6$ Alkylgruppe; eine Halogen-$C_1$-$C_6$ Alkylgruppe; eine $C_3$-$C_6$ Alkenylgruppe; eine Halogen-$C_3$-$C_6$ Alkenylgruppe; eine $C_3$-$C_6$ Alkinylgruppe; eine Halogen-$C_3$-$C_6$ Alkinylgruppe; eine $C_3$-$C_6$ Cycloalkylgruppe, eine Halogen-$C_3$-$C_6$ Cycloalkylgruppe; eine $C_1$-$C_6$ Alkylcarbonylgruppe; eine Halogen-$C_1$-$C_6$ Alkylcarbonylgruppe; eine $C_1$-$C_6$ Alkoxycarbonylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; einer Phenyl-$C_1$-$C_4$ Alkylgruppe; einer substituierten Phenyl-$C_1$-$C_4$ Alkylgruppe mit mindestens einem Substituenten im Phenylring, welcher gleich oder verschieden

sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, eine $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; einer heterocyclischen Gruppe (die wie unten definiert ist); oder einer substituierten heterocyclischen Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_{1-}C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe), und

I ist eine ganze Zahl von 1 bis 4);

weiterhin können $R^1$ und $R^2$ einen 4-7-gliedrigen Ring bilden, indem sie miteinander kombiniert werden, und der Ring kann die gleichen oder verschiedene 1 bis 3 Heteroatome ausgewählt aus der Gruppe bestehend aus Sauerstoffatom, Schwefelatom und Stickstoffatom enthalten;

$X^1$ und $X^2$ können gleich oder verschieden sein und jedes ist ein Halogenatom; eine Cyanogruppe; eine $C_1$-$C_6$ Alkylgruppe; eine Halogen-$C_1$-$C_6$ Alkylgruppe; eine Halogen-$C_1$-$C_6$ Alkoxygruppe; eine $C_1$-$C_6$ Alkylthiogruppe; eine Halogen-$C_1$-$C_6$ Alkylthiogruppe; eine $C_1$-$C_6$ Alkylsulfinylgruppe; eine Halogen-$C_1$-$C_6$ Alkylsunlfinylgruppe; eine $C_1$-$C_6$ Alkylsulfonylgruppe und eine Halogen-$C_1$-$C_6$ Alkylsulfonylgruppe ist; weiterhin,

$X^1$ und $X^2$ können einen anellierten Ring mit dem Phenylring des Phthalsäurediamidderivats bilden (dies bedeutet Naphthalin, Tetrahydronaphthalin, Inden, Indan, Chinolin, Chinazolin, Chroman, Isochroman, Indol, Indolin, Benzodioxan, Benzodioxol, Benzofuran, Dihydrobenzofuran, Benzothiophen, Dihydrobenzothiophen, Benzoxazol, Benzothiazol, Benzimidazol oder Indazol), indem sie miteinander kombiniert werden, und dieser anellierte Ring kann mindestens einen Substituenten tragen, der gleich oder verschieden sein kann und ausgewählt ist aus der Gruppe bestehend aus einem Halogenatom; einer $C_1$-$C_6$ Alkylgruppe; einer Halogen-$C_1$-$C_6$ Alkylgruppe; einer $C_1$-$C_6$ Alkoxygruppe; einer Halogen-$C_1$-$C_6$ Alkoxygruppe; einer $C_1$-$C_6$ Alkylthiogruppe; einer Halogen-$C_1$-$C_6$ Alkylthiogruppe; einer $C_1$-$C_6$ Alkylsulfinylgruppe; einer Halogen-$C_1$-$C_6$ Alkylsulfinylgruppe; einer $C_1$-$C_6$ Alkylsulfonylgruppe; einer Halogen-$C_1$-$C_6$ Alkylsulfonylgruppe; einer Phenylgruppe; einer substituierten Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; einer heterocyclischen Gruppe (die wie oben definiert ist) und einer substituierten heterocyclischen Gruppe (worin die heterocyclische Gruppe wie oben definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_{1-}C_6$ Alkylsulfonylgruppe und einer Halogen-$C_1$-$C_6$ Alkylsulfonylgruppe;

Y ist gleich oder verschieden, und jedes ist ein Wasserstoffatom; ein Halogenatom; eine Cyanogruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclische Gruppe (die wie oben definiert ist); eine substituierte heterocyclische Gruppe (worin die heterocyclische Gruppe wie oben definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; oder eine Gruppe —$A^2$-$R^7$ (worin $A^2$ —O-; -S-; -SO-; $SO_2$; - C(=O)-; -C(=NOR$^8$)- ist (worin $R^8$ ein Wasserstoffatom ist; eine $C_1$-$C_6$ Alkylgruppe; ein Halogen-$C_1$-$C_6$ Alkylgruppe; eine $C_3$-$C_6$ Alkenylgruppe; eine Halogen-$C_3$-$C_6$ Alkenylgruppe; eine $C_3$-$C_6$ Alkinylgruppe; eine $C_3$-$C_6$ Cycloalkylgruppe; eine Phenyl-$C_1$-$C_4$ Alkylgruppe; oder eine substituierte Phenyl-$C_1$-$C_4$ Alkylgruppe mit mindestens einem Substituenten im Phenylring, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinyl-

gruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe); eine $C_1$-$C_6$ Alkylengruppe, eine Halogen-$C_1$-$C_6$ Alkylengruppe; eine $C_2$-$C_6$ Alkenylengruppe; eine Halogen-$C_2$-$C_6$ Alkenylengruppe; eine $C_2$-$C_6$ Alkinylengruppe oder eine Halogen-$C_3$-$C_6$ Alkinylengruppe;

(1) wenn $A^2$ -O-; -S-; -SO- oder -$SO_2$- ist, dann ist $R^7$ eine Halogen-$C_3$-$C_6$ Cycloalkylgruppe; eine Halogen-$C_3$-$C_6$ Cycloalkenylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclischen Gruppe (die wie unten definiert ist), eine substituierten heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_{1\text{-}}C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; oder eine Gruppe der Formel -$A^3$-$R^9$
(worin $A^3$ eine $C_1$-$C_6$ Alkylengruppe, eine Halogen-$C_1$-$C_6$ Alkylengruppe, eine $C_{3\text{-}}C_6$ Alkenylengruppe, eine Halogen-$C_3$-$C_6$ Alkenylengruppe, eine $C_3$-$C_9$ Alkinylengruppe oder eine Halogen-$C_3$-$C_6$ Alkinylengruppe ist; $R^9$ ist ein Wasserstoffatom; ein Halogenatom; eine $C_3$-$C_6$ Cycloalkylgruppe, eine Halogen-$C_3$-$C_6$ Cycloalkylgruppe; eine $C_1$-$C_6$ Alkoxycarbonylgruppe; eine Phenylgruppe, eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe; einer Halogen-$C_{1\text{-}}C_6$ Alkylgruppe; einer $C_1$-$C_6$ Alkoxygruppe; einer Halogen-$C_1$-$C_6$ Alkoxygruppe; einer $C_1$-$C_6$ Alkylthiogruppe; einer Halogen-$C_1$-$C_6$ Alkylthiogruppe; einer $C_1$-$C_6$ Alkylsulfinylgruppe; einer Halogen-$C_1$-$C_6$ Alkylsulfinylgruppe; einer $C_1$-$C_6$ Alkylsulfonylgruppe; einer Halogen-$C_1$-$C_6$ Alkylsulfonylgruppe oder einer Gruppe der Formel -$A^4$-$R^{10}$
(worin $A^4$ -O-; -S-; -SO-; -$SO_2$- oder -C(=O)- ist, und
$R^{10}$ eine $C_1$-$C_6$ Alkylgruppe; eine Halogen-$C_1$-$C_6$ Alkylgruppe; eine $C_3$-$C_6$ Alkenylgruppe; eine Halogen-$C_3$-$C_6$ Alkenylgruppe; eine $C_3$-$C_6$ Cycloalkylgruppe; eine Halogen-$C_3$-$C_6$ Cycloalkylgruppe; eine Phenylgruppe, eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclische Gruppe (die wie unten definiert ist); oder eine substituierte heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_8$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe ist));
(2) wenn $A^2$ -C(=O)- oder eine Gruppe der Formel -C(=NOR$^8$)- ist (worin $R^8$ wie oben definiert ist), dann ist $R^7$ eine $C_1$-$C_6$ Alkylgruppe; eine Halogen $C_1$-$C_6$ Alkylgruppe; eine $C_2$-$C_6$ Alkenylgruppe; eine Halogen-$C_2$-$C_6$ Alkenylgruppe; eine $C_{3\text{-}}C_6$ Cycloalkylgruppe; eine Halogen-$C_3$-$C_6$ Cycloalkylgruppe; eine $C_1$-$C_6$ Alkoxygruppe; eine $C_1$-$C_6$ Alkylthiogruppe; eine Mono-$C_1$-$C_6$ Alkylaminogruppe; eine Di-$C_1$-$C_6$ Alkylaminogruppe, welche gleich oder verschieden sein kann; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$, Alkylgruppe, einer Halogen-$C_{1\text{-}}C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine Phenylaminogruppe; eine substituierte Phenylaminogruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_{1\text{-}}C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Al-

kylsulfonylgruppe; eine heterocyclische Gruppe (die wie unten definiert ist), oder eine substituierte heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$Alkylthiogruppe, einer $C_1$-$C_6$ Alkylsulfinylgruppe, einer Halogen-$C_1$-$C_6$ Alkylsulfinylgruppe, einer $C_{1-}C_6$ Alkylsulfonylgruppe und einer Halogen-$C_1$-$C_6$ Alkylsulfonylgruppe;

(3) wenn $A^2$ eine $C_1$-$C_6$ Alkylengruppe; eine Halogen-$C_1$-$C_6$ Alkylengruppe; $C_2$-$C_6$ Alkenylengruppe; eine Halogen-$C_2$-$C_6$ Alkenylengruppe; eine $C_2$-$C_6$ Alkinylengruppe oder eine Halogen-$C_3$-$C_6$ Alkinylengruppe ist; dann ist $R^7$ ein Wasserstoffatom; ein Halogenatom; eine $C_3$-$C_6$ Cycloalkylgruppe; eine Halogen-$C_3$-$C_6$ Cycloalkylgruppe; eine $C_1$-$C_6$ Alkoxycarbonylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_{1-}C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclische Gruppe (die wie unten definiert ist); eine substituierte heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, eine $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; oder eine Gruppe der Formel -$A^5$-$R^{12}$

(worin $A^5$ -O-; -S-; -SO- oder-$SO_2$- ist, und

$R^{12}$ ist eine $C_3$-$C_6$ Cycloalkylgruppe; eine Halogen-$C_3$-$C_6$ Cycloalkylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe, eine heterocyclische Gruppe (die wie unten definiert ist); eine substituierte heterocyclische Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; oder eine Gruppe der Formel -$A^6$-$R^{14}$

(worin $A^6$ eine $C_1$-$C_6$ Alkylengruppe, eine Halogen-$C_1$-$C_6$ Alkylengruppe, eine $C_{2-}C_6$ Alkenylengruppe, eine Halogen-$C_2$-$C_6$ Alkenylengruppe, eine $C_2$-$C_6$ Alkinylengruppe oder eine Halogen-$C_3$-$C_6$ Alkinylengruppe ist; und

$R^{14}$ ein Wasserstoffatom ist; ein Halogenatom; eine $C_3$-$C_6$ Cycloalkylgruppe; eine Halogen-$C_3$-$C_6$ Cycloalkylgruppe; eine $C_1$-$C_6$ Alkoxygruppe; eine Halogen-$C_1$-$C_6$ Alkoxygruppe; eine $C_1$-$C_6$ Alkylthiogruppe; eine Halogen-$C_1$-$C_6$ Alkylthiogruppe; eine $C_1$-$C_6$ Alkylsulfinylgruppe; eine Halogen-$C_1$-$C_6$ Alkylsulfinylgruppe; eine $C_1$-$C_6$ Alkylsulfonylgruppe; eine Halogen-$C_1$-$C_6$ Alkylsulfonylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine Phenoxygruppe, eine substituierte Phenoxygruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine Phenylthiogruppe, eine substituierte Phenylthiogruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-

$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; eine heterocyclische Gruppe (die wie unten definiert ist); oder eine substituierte heterocyclische Gruppe (worin der heterocyclische Ring wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe))); und

m ist eine ganze Zahl von 1 bis 5;
weiterhin kann Y einen anellierten Ring bilden (der wie oben definiert ist), indem es mit den benachbarten Kohlenstoffatomen im Phenylring kombiniert wird, wobei der anellierte Ring mindestens einen Substituenten haben kann, welcher gleich oder verschieden sein kann und ausgewählt ist aus der Gruppe bestehend aus einem Halogenatom; einer $C_1$-$C_6$ Alkylgruppe; einer Halogen-$C_1$-$C_6$ Alkylgruppe; einer $C_1$-$C_6$ Alkoxygruppe; einer Halogen-$C_1$-$C_6$ Alkoxygruppe; einer $C_1$-$C_6$ Alkylthiogruppe; einer Halogen-$C_1$-$C_6$ Alkylthiogruppe; einer $C_1$-$C_6$ Alkylsulfinylgruppe; einer Halogen-$C_1$-$C_6$ Alkylsulfinylgruppe; einer $C_1$-$C_6$ Alkylsulfonylgruppe; einer Halogen-$C_1$-$C_6$ Alkylsulfonylgruppe; einer Phenylgruppe; einer substituierten Phenylgruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe; einer heterocyclische Gruppe (die wie unten definiert ist); und einer substituierten heterocyclischen Gruppe (worin die heterocyclische Gruppe wie unten definiert ist) mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe, einer $C_1$-$C_6$ Alkylsulfonylgruppe und einer Halogen-$C_1$-$C_6$ Alkylsulfonylgruppe;
$Z^1$ und $Z^2$ jeweils ein Sauerstoffatom oder ein Schwefelatom sind; und

die heterocyclische Gruppe ausgewählt ist aus der Gruppe bestehend aus einer Pyridylgruppe, Pyridin-N-Oxidgruppe, Pyrimidinylgruppe, Furylgruppe, Tetrahydrofurylgruppe, Thienylgruppe, Tetrahydrothienylgruppe, Tetrahydropyranylgruppe, Tetrahydrothiopyranylgruppe, Oxazolylgruppe, Isoxazolylgruppe, Oxadiazolylgruppe, Thiazolylgruppe, Isothiazolylgruppe, Thiadiazolylgruppe, Imidazolylgruppe, Triazolylgruppe und Pyrazolylgruppe}.

**2.** Ein Phthalsäurediamidderivat gemäß Anspruch 1, dargestellt durch die allgemeine Formel (I-3),

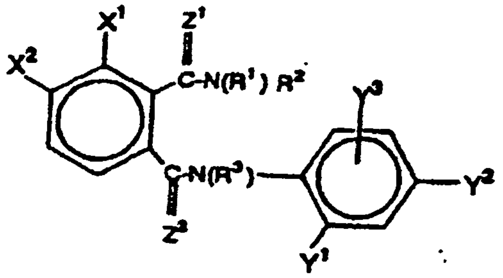

(I-3)

worin $R^1$, $R^2$ und $R^3$, l, $X^1$ und $X^2$ wie in Anspruch 1 für die Formel (1-2) definiert sind;
$Y^1$ und $Y^3$ können gleich oder verschieden sein und jedes ein Wasserstoffatom sein; ein Halogenatom; eine $C_1$-$C_6$ Alkylgruppe; eine Halogen-$C_1$-$C_6$ Alkylgruppe; eine $C_1$-$C_6$ Alkoxygruppe; eine Halogen-$C_1$-$C_6$ Alkoxygruppe; eine $C_1$-$C_6$ Akylthiogruppe; eine Halogen-$C_1$-$C_6$ Alkylthiogruppe; eine Phenoxygruppe; eine substituierte Phenoxygruppe mit mindestens einem Substituenten, welcher gleich oder verschieden sein kann und ausgewählt ist aus der Gruppe bestehend aus einem Halogenatom, einer $C_1$-$C_6$ Alkylgruppe, einer Halogen-$C_1$-$C_6$ Alkylgruppe, einer $C_1$-$C_6$ Alkoxygruppe, einer Halogen-$C_1$-$C_6$ Alkoxygruppe, einer $C_1$-$C_6$ Alkylthiogruppe, einer Halogen-$C_1$-$C_6$ Alkylthiogruppe, einer $C_1$-$C_6$ Alkylsulfinylgruppe, einer Halogen-$C_1$-$C_6$ Alkylsulfinylgruppe,

einer $C_1$-$C_6$ Alkylsulfonylgruppe und einer Halogen-$C_1$-$C_6$ Alkylsulfonylgruppe; eine Pyridyloxygruppe; oder eine substituierte Pyridyloxygruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer Halogen-$C_1$-$C_6$ Alkylsulfinylgruppe und einer Halogen-$C_1$-$C_6$ Alkylsulfonylgruppe,

$Y^2$ ist ein Wasserstoffatom; ein Halogenatom oder eine Gruppe der Formel $-A^2-R^7$

(worin $A^2$ -O-; -S-; -SO-; $-SO_2-$; eine $C_1$-$C_6$ Alkylengruppe; eine Halogen-$C_1$-$C_6$ Alkylengruppe; eine $C_2$-$C_6$ Alkenylengruppe; eine Halogen-$C_2$-$C_6$ Alkenylengruppe; eine $C_2$-$C_6$ Alkinylengruppe oder eine Halogen-$C_3$-$C_6$ Alkinylengruppe ist; und

(1) wenn $A^2$ -O-; -S-; -SO- oder $-SO_2-$ ist, dann ist $R^7$ eine Halogen-$C_3$-$C_6$ Cycloalkylgruppe; eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; eine substituierte Pyridyloxygruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; oder eine Gruppe der Formel $-A^3-R^9$

(worin $A^3$ eine Halogen-$C_1$-$C_6$ Alkylengruppe oder eine Halogen-$C_3$-$C_6$ Alkenylengruppe ist und;

$R^9$ ist ein Wasserstoffatom; ein Halogenatom; eine Halogen-$C_3$-$C_6$ Cycloalkylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; oder eine Gruppe der Formel $-A^4-R^{10}$

(worin $A^4$ -O-; -S-; -SO- oder $-SO_2-$ ist;

$R^{10}$ ist eine $C_1$-$C_6$ Alkylgruppe; eine Halogen $C_1$-$C_6$ Alkylgruppe; eine $C_3$-$C_6$ Alkenylgruppe; eine Halogen-$C_3$-$C_6$ Alkenylgruppe; eine $C_3$-$C_6$ Cycloalkylgruppe; eine Halogen-$C_3$-$C_6$ Cycloalkylgruppe; eine Phenylgruppe; oder eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe));

(2) wenn $A^2$ eine $C_1$-$C_6$ Alkylengruppe; eine Halogen-$C_1$-$C_6$ Alkylengruppe; eine $C_2$-$C_6$ Alkenylengruppe; eine Halogen-$C_2$-$C_6$ Alkenylengruppe; eine $C_2$-$C_6$ Alkinylengruppe; eine Halogen-$C_3$-$C_6$ Alkinylengruppe ist; dann ist $R^7$ ein Wasserstoffatom; ein Halogenatom; eine Halogen-$C_3$-$C_6$ Cycloalkylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; oder eine Gruppe der Formel $-A^5-R^{12}$

(worin $A^5$ -O-; -S-; -SO- oder $-SO_2-$ ist und

$R^{12}$ ist eine $C_3$-$C_6$ Cycloalkylgruppe; eine Halogen-$C_3$-$C_6$ Cycloalkylgruppe; eine Phenylgruppe; eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; oder eine Gruppe der Formel $-A^6-R^{14}$

(worin $A^6$ eine $C_1$-$C_6$ Alkylengruppe; eine Halogen-$C_1$-$C_6$ Alkylengruppe; eine $C_2$-$C_6$ Alkenylengruppe; eine Halogen-$C_2$-$C_6$ Alkenylengruppe ist; und

$R^{14}$ ist ein Wasserstoffatom; ein Halogenatom; eine Halogen-$C_3$-$C_6$ Cycloalkylgruppe; eine Halogen-$C_1$-$C_6$ Alkoxygruppe; eine Halogen-$C_1$-$C_6$ Alkylthiogruppe; eine Halogen-$C_1$-$C_6$ Alkylsulfinylgruppe; eine Halogen-$C_1$-$C_6$ Alkylsulfonylgruppe; eine Phenylgruppe, eine substituierte Phenylgruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$Alkylsulfinylgruppe und einer Halogen-$C_1$-$C_6$ Alkylsulfonylgruppe; eine Phenoxygruppe; eine substituierte Phenoxygruppe mit mindestens einem Substituenten, welcher gleich oder verschieden sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe; eine Phenylthiogruppe; oder eine substituierte Phenylthiogruppe mit mindestens einem Substituenten, welcher gleich oder verschieden

sein kann und ausgewählt ist aus der Gruppe bestehend aus einem Halogenatom, einer Halogen-$C_1$-$C_6$ Alkylgruppe, einer Halogen-$C_1$-$C_6$ Alkoxygruppe, einer Halogen-$C_1$-$C_6$ Alkylthiogruppe, einer $C_1$-$C_6$ Alkylsulfinylgruppe und einer Halogen-$C_1$-$C_6$ Alkylsulfonylgruppe)); und weiterhin,

$Z^1$ und $Z^2$ wie in Anspruch 1 beschrieben sind mit Hinblick auf Formel (I-2).

**3.** Verwendung des Phthalsäurediamidderivats gemäß Anspruch 1 oder 2 als landwirtschaftliches und gartenbautechnisches Insektizid.

**4.** Verfahren zur Kontrolle von unerwünschten Insektenschädlingen bei Nutzgetreide, **dadurch gekennzeichnet, dass** das betreffende Getreide mit einer wirksamen Menge des Phthalsäurediamidderivats gemäß Anspruch 1 oder 2 behandelt wird.

**5.** Ein landwirtschaftliches und gartenbautechnisches Insektizid, umfassend das Phthalsäurediamidderivat gemäß Anspruch 1 oder 2 als aktive Komponente.

## Revendications

**1.** Dérivé diamide de l'acide phtalique, représenté par la formule générale (I-2)

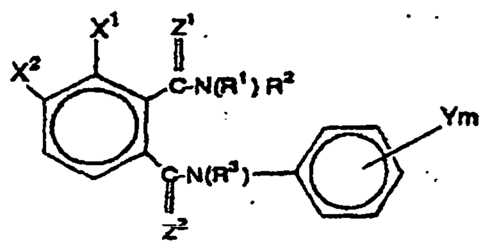

(I-2)

{dans laquelle

$R^1$, $R^2$ et $R^3$ peuvent être identiques ou différents, et sont chacun un atome d'hydrogène ; un groupe cycloalkyle en $C_3$-$C_6$ ; un groupe halogéno-cycloalkyle en $C_3$-$C_6$ ou un groupe de formule -$A^1$-$Q_l$ (où $A^1$ est un groupe alkylène en $C_1$-$C_8$ ; un groupe alcénylène en $C_3$-$C_6$ ou un groupe alcynylène en $C_3$-$C_6$ ;
Q est un atome d'hydrogène ; un atome d'halogène ; un groupe cyano ; un groupe nitro ; un groupe halogéno-alkyle en $C_1$-$C_6$ ; un groupe cycloalkyle en $C_3$-$C_6$ ; un groupe halogéno-cycloalkyle en $C_3$-$C_6$ ; un groupe alcoxy en $C_1$-$C_6$-carbonyle ; un groupe di-alcoxy en $C_1$-$C_6$-phosphoryle qui peut être identique ou différent ; un groupe di-alcoxy en $C_1$-$C_6$-thiophosphoryle qui peut être identique ou différent ; un groupe diphénylphosphino ; un groupe diphénylphosphono ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (dans lequel le groupe hétérocyclique est défini comme ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; ou un groupe de formule -$Z^3$-$R^5$ (où $Z^3$ est -O- ; -S- ; -SO- ; -$SO_2$- ou un groupe de formule -N($R^6$)-(où $R^6$ est un atome d'hydrogène ; un groupe alkyl en $C_1$-$C_6$-carbonyle ; un groupe halogéno-alkyl en $C_1$-$C_6$-carbonyle ; un groupe alcoxy en $C_1$-$C_6$-carbonyle ; un groupe phénylcarbonyle ; un groupe phénylcarbonyle substitué ayant au moins un substituant qui peut être identique ou différent et est choisi dans le groupe constitué par un atome d'halogène ; un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un

groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe phényl alcoxy en $C_1$-$C_4$-carbonyle ou un groupe phényl alcoxy en $C_1$-$C_4$-carbonyle substitué ayant au moins un substituant, dans le noyau phényle, qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle) ; et

$R^5$ est un atome d'hydrogène ; un groupe alkyle en $C_1$-$C_6$ ; un groupe halogéno-alkyle en $C_1$-$C_6$ ; un groupe alcényle en $C_3$-$C_6$; un groupe halogéno-alcényle en $C_3$-$C_6$ ; un groupe alcynyle en $C_3$-$C_6$ ; un groupe halogéno-alcynyle en $C_3$-$C_6$ ; un groupe cycloalkyle en $C_3$-$C_6$ ; un groupe halogéno-cycloalkyle en $C_3$-$C_6$ ; un groupe alkyl en $C_1$-$C_6$-carbonyle ; un groupe halogéno-alkyl en $C_1$-$C_6$-carbonyle ; un groupe alcoxy en $C_1$-$C_6$-carbonyle ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe phényl alkyle en $C_1$-$C_4$ ; un groupe phényl alkyle en $C_1$-$C_4$ substitué ayant au moins un substituant, dans le noyau phényle, qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$ ; un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle) ; et

1 est un entier de 1 à 4) ; en outre,

$R^1$ et $R^2$ peuvent former un noyau de 4 à 7 chaînons en se combinant l'un à l'autre, le noyau pouvant contenir les 1 à 3 hétéroatomes identiques ou différents choisis dans le groupe constitué par l'atome d'oxygène, l'atome de soufre et l'atome d'azote ;

$X^1$ et $X^2$ peuvent être identiques ou différents et sont chacun un atome d'halogène ; un groupe cyano ; un groupe alkyle en $C_1$-$C_6$ ; un groupe halogéno-alkyle en $C_1$-$C_6$ ; un groupe halogéno-alcoxy en $C_1$-$C_6$ ; un groupe alkylthio en $C_1$-$C_6$ ; un groupe halogéno-alkylthio en $C_1$-$C_6$ ; un groupe alkyl en $C_1$-$C_6$-sulfinyle ; un groupe halogéno-alkyl en $C_1$-$C_6$-sulfinyle : un groupe alkyl en $C_1$-$C_6$-sulfonyle et un groupe halogéno-alkyl en $C_1$-$C_6$-sulfonyle ; en outre,

$X^1$ et $X^2$ peuvent former un noyau condensé avec le noyau phényle du dérivé amide de l'acide phtalique (ce qui signifie naphtalène, tétrahydrcnaphtalène, indène, indane, quinoléine, quinazoline, chromane, isochromane, indole, indoline, benzodioxane, benzodioxole, benzofuranne, dihydrobenzofuranne, benzothiophène, dihydrobenzothiophène, benzoxazole, benzothiazole, benzimidazole ou indazole) en se combinant l'un à l'autre, et ledit noyau condensé peut avoir au moins un substituant, qui peut être identique ou différent et est choisi dans le groupe constitué par un atome d'halogène ; un groupe alkyle en $C_1$-$C_6$ ; un groupe halogéno-alkyle en $C_1$-$C_6$ ; un groupe alcoxy en $C_1$-$C_6$ ; un groupe halogéno-alcoxy en $C_1$-$C_6$ ; un groupe alkylthio en $C_1$-$C_6$ ; un groupe halogéno-alkylthio en $C_1$-$C_6$ ; un groupe alkyl en $C_1$-$C_6$-sulfinyle ; un groupe halogéno-alkyl en $C_1$-$C_6$-sulfinyle ; un groupe alkyl en $C_1$-$C_6$-sulfonyle ; un groupe halogéno-alkyl en $C_1$-$C_6$-sulfonyle ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; et un groupe hétérocyclique substitué (dans lequel le groupe hétérocyclique est le même que celui défini ci-dessus) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ;

Y est identique ou différent, et sont chacun un atome d'hydrogène ; un atome d'halogène ; un groupe cyano ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; ou un groupe $-A^2$-$R^7$- (où $A^2$ est -O- ; -S- ; -SO- ; $-SO_2$- -C(=O)- ; -C(=$NOR^8$)- (où $R^8$ est un atome d'hydrogène ; un groupe alkyle en $C_1$-$C_6$ ; un groupe halogéno-alkyle en $C_1$-$C_6$ ; un groupe alcényle en $C_3$-$C_6$ ; un groupe halogéno-alcényle en $C_3$-$C_6$ ; un groupe alcynyle en $C_3$-$C_6$ ; un groupe cycloalkyle en $C_3$-$C_6$ ; un groupe phényl-alkyle en $C_1$-$C_4$ ; ou un groupe phényl-alkyle en $C_1$-$C_4$ substitué ayant au moins un substituant, dans le noyau phényle, qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle) ; un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; un groupe alcynylène en $C_2$-$C_6$ ou un groupe halogéno-alcynylèné en $C_3$-$C_6$ ;

(1) lorsque $A^2$ est -O- ; -S- ; -SO- ou $-SO_2$-, alors $R^7$ est un groupe halogéno-cycloalkyle en $C_3$-$C_6$ ; un groupe halogéno-cycloalcényle en $C_3$-$C_6$ ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; ou un groupe de formule $-A^3$-$R^9$ (où $A^3$ est un groupe alkylène en $C_1$-$C_6$ ; un groupe halogéno-alkylène en $C_1$-$C_6$ ; un groupe alcénylène en $C_3$-$C_6$ ; un groupe halogéno-alcénylène en $C_3$-$C_6$ ; un groupe alcynylène en $C_3$-$C_6$ ou un groupe halogéno-alcynylène en $C_3$-$C_6$ ;
$R^9$ est un atome d'hydrogène ; un atome d'halogène ; un groupe cycloalkyle en $C_3$-$C_6$ ; un groupe halogéno-cycloalkyle en $C_3$-$C_6$ ; un groupe alcoxy en $C_1$-$C_6$-carbonyle ; un groupe phényle ; un groupe phényle substitué ayant au moins un substituant qui peut être identique ou différent et est choisi dans le groupe constitué par un atome d'halogène ; un groupe alkyle en $C_1$-$C_6$ ; un groupe halogéno-alkyle en $C_1$-$C_6$ ; un groupe alcoxy en $C_1$-$C_6$ ; un groupe halogéno-alcoxy en $C_1$-$C_6$ ; un groupe alkylthio en $C_1$-$C_6$ ; un groupe halogéno-alkylthio en $C_1$-$C_6$ : un groupe alkyl en $C_1$-$C_6$-sulfinyle ; un groupe halogéno-alkyl en $C_1$-$C_6$-sulfinyle ; un groupe alkyl en $C_1$-$C_6$-sulfonyle ; un groupe halogéno-alkyl en $C_1$-$C_6$-sulfonyle ou un groupe de formule $-A^4$-$R^{10}$ (où $A^4$ est -O- ; -S- ; -SO- ; $-SO_2$- ou -C (=O) -, et $R^{10}$ est un groupe alkyle en $C_1$-$C_6$ ; un groupe halogéno-alkyle en $C_1$-$C_6$ ; un groupe alcényle en $C_3$-$C_6$ ; un groupe halogéno-alcényle en $C_3$-$C_6$ ; un groupe cycloalkyle en $C_3$-$C_6$ ; un groupe halogéno-cycloalkyle en $C_3$-$C_6$ ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe

alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle)) ;

(2) lorsque $A^2$ est -C(=O)- ou un groupe de formule -C(=NOR$^8$)- (où $R^8$ est le même que celui défini ci-dessus), alors $R^7$ est un groupe alkyle en $C_1$-$C_6$ ; un groupe halogéno-alkyle en $C_1$-$C_6$ ; un groupe alcényle en $C_2$-$C_6$ ; un groupe halogéno-alcényle en $C_2$-$C_6$ ; un groupe cycloalkyle en $C_3$-$C_6$ ; un groupe halogéno-cycloalkyle en $C_3$-$C_6$ ; un groupe alcoxy en $C_1$-$C_6$ ; un groupe alkylthio en $C_1$-$C_6$ ; un groupe mono-alkyl en $C_1$-$C_6$-amino ; un groupe di-alkyl en $C_1$-$C_6$-amino qui peut être identique ou différent ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe phénylamino ; un groupe phénylamino substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinye, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; ou un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ;

(3) lorsque $A^2$ est un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; un groupe alcynylène en $C_2$-$C_6$ ou un groupe halogéno-alcynylène en $C_3$-$C_6$ ; alors $R^7$ est un atome d'hydrogène ; un atome d'halogène ; un groupe cycloalkyle en $C_3$-$C_6$; un groupe halogéno-cycloalkyle en $C_3$-$C_6$; un groupe alcoxy en $C_1$-$C_6$-carbonyle ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; ou un groupe de formule -$A^5$-$R^{12}$ (où $A^5$ est -O- ; -S- ; -SO- ou -$SO_2$- ; et $R^{12}$ est un groupe cycloalkyle en $C_3$-$C_6$ ; un groupe halogéno-cycloalkyle en $C_3$-$C_6$ ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; ou un groupe de formule -$A^6$-$R^{14}$ (où $A^6$ est un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; un groupe alcynylène en $C_2$-$C_6$ ; ou un groupe halogéno-alcynylène en $C_3$-$C_6$ ; et $R^{14}$ est un atome d'hydrogène ; un atome d'halogène ; un groupe cycloalkyle en $C_3$-$C_6$ ; un groupe halogéno-cycloalkyle en $C_3$-$C_6$ ; un groupe alcoxy en $C_1$-$C_6$ ; un groupe halogéno-alcoxy en $C_1$-$C_6$ ; un groupe alkylthio

en $C_1$-$C_6$ ; un groupe halogéno-alkylthio en $C_1$-$C_6$ ; un groupe alkyl en $C_1$-$C_6$-sulfinyle ; un groupe halogéno-alkyl en $C_1$-$C_6$-sulfinyle ; un groupe alkyl en $C_1$-$C_6$-sulfonyle ; un groupe halogéno-alkyl en $C_1$-$C_6$-sulfonyle ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe phénoxy ; un groupe phénoxy substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe phénylthio : un groupe phénylthio substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; ou un groupe hétérocyclique substitué (dans lequel le noyau hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle))); et

m est un entier de 1 à 5 ;

en outre, Y peut former un noyau condensé (qui est le même que celui défini ci-dessus) en se combinant avec les atomes de carbone adjacents dans le noyau phényle, ledit noyau condensé peut avoir au moins un substituant, qui est identique ou différent et est choisi dans le groupe constitué par un atome d'halogène ; un groupe alkyle en $C_1$-$C_6$ ; un groupe halogéno-alkyle en $C_1$-$C_6$ ; un groupe alcoxy en $C_1$-$C_6$ ; un groupe halogéno-alcoxy en $C_1$-$C_6$ ; un groupe alkylthio en $C_1$-$C_6$ ; un groupe halogéno-alkylthio en $C_1$-$C_6$ ; un groupe alkyl en $C_1$-$C_6$-sulfinyle ; un groupe halogéno-alkyl en $C_1$-$C_6$-sulfinyle ; un groupe alkyl en $C_1$-$C_6$-sulfonyle ; un groupe halogéno-alkyl en $C_1$-$C_6$-sulfonyle ; un groupe phényle ; un groupe phényle substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe hétérocyclique (qui est le même que celui défini ci-après) ; et un groupe hétérocyclique substitué (dans lequel le groupe hétérocyclique est le même que celui défini ci-après) ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ;

$Z^1$ et $Z^2$ sont chacun un atome d'oxygène ou un atome de soufre ; et

ledit groupe hétérocyclique est choisi dans le groupe constitué par un groupe pyridyle, un groupe pyridine-N-oxyde, un groupe pyrimidinyle, un groupe furyle, un groupe tétrahydrofuryle, un groupe thiényle, un groupe tétrahydrothiényle, un groupe tétrahydropyranyle, un groupe tétrahydrothiopyranyle, un groupe oxazolyle, un groupe isoxazolyle, un groupe oxadiazolyle, un groupe thiazolyle, un groupe isothiazolyle, un groupe thiadiazolyle, un groupe imidazolyle, un groupe triazolyle et un groupe pyrazolyle}.

**2.** Dérivé amide de l'acide phtalique selon la revendication 1, représenté par la formule générale (I-3),

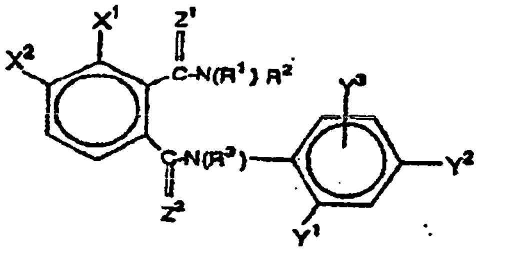

(I-3)

{dans laquelle $R^1$, $R^2$ et $R^3$, l, $X^1$ et $X^2$ sont tels que définis dans la revendication 1 en ce qui concerne la formule (I-2) ;

$Y^1$ et $Y^3$ peuvent être identiques ou différents et sont chacun un atome d'hydrogène ; un atome d'halogène ; un groupe alkyle en $C_1$-$C_6$ ; un groupe halogéno-alkyle en $C_1$-$C_6$ ; un groupe alcoxy en $C_1$-$C_6$; un groupe halogéno-alcoxy en $C_1$-$C_6$; un groupe alkylthio en $C_1$-$C_6$ ; un groupe halogéno-alkylthio en $C_1$-$C_6$ ; un groupe phénoxy ; un groupe phénoxy substitué ayant au moins un substituant qui peut être identique ou différent et est choisi dans le groupe constitué par un atome d'halogène, un groupe alkyle en $C_1$-$C_6$, un groupe halogéno-alkyle en $C_1$-$C_6$, un groupe alcoxy en $C_1$-$C_6$, un groupe halogéno-alcoxy en $C_1$-$C_6$, un groupe alkylthio en $C_1$-$C_6$, un groupe halogéno-alkylthio en $C_1$-$C_6$, un groupe alkyl en $C_1$-$C_6$-sulfinyle, un groupe halogéno-alkyl en $C_1$-$C_6$-sulfinyle, un groupe alkyl en $C_1$-$C_6$-sulfonyle et un groupe halogéno-alkyl en $C_1$-$C_6$-sulfonyle ; un groupe pyridyloxy ; ou un groupe pyridyloxy substitué ayant au moins un substituant qui peut être identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_{6}$-sulfinyle et un groupe halogéno-alkyl en $C_1$-$C_{6}$-sulfonyle ;

$Y^2$ est un atome d'hydrogène ; un atome d'halogène ou un groupe de formule -$A^2$-$R^7$ (où $A^2$ est -O- ; -S- ; -SO- ; -$SO_2$- ; un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; un groupe alcynylène en $C_2$-$C_6$ ; ou un groupe halogéno-alcynylène en $C_3$-$C_6$ ; et

(1) lorsque $A^2$ est -O- ; -S- ; -SO- ou -$SO_2$-, alors $R^7$ est un groupe halogéno-cycloalkyle en $C_3$-$C_6$ ; un groupe phényle substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe alkyl en $C_1$-$C_6$-sulfinyle et un groupe halogéno-alkyl en $C_1$-$C_6$-sulfonyle ; un groupe pyridyloxy substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_{6}$-sulfinyle et un groupe halogéno-alkyl en $C_1$-$C_{6}$-sulfonyle ; ou un groupe de formule -$A^3$-$R^9$ (où $A^3$ est un groupe halogéno-alkylène en $C_1$-$C_6$ ou un groupe halogéno-alcénylène en $C_3$-$C_6$ et ; $R^9$ est un atome d'hydrogène ; un atome d'halogène ; un groupe halogéno-cycloalkyle en $C_3$-$C_6$ ; un groupe phényle ; un groupe phényle substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_{6}$-sulfinyle et un groupe rtalogéno-aikyl en $C_1$-$C_{6}$-sulfonyle ; ou un groupe de formule -$A^4$-$R^{10}$ (où $A^4$ est -O- ; -S- ; -SO- ou -$SO_2$- ; $R^{10}$ est un groupe alkyle en $C_1$-$C_6$ ; un groupe halogéno-alkyle en $C_1$-$C_6$ ; un groupe alcényle en $C_3$-$C_6$; un groupe halogéno-alcényle en $C_3$-$C_6$ ; un groupe cycloalkyle en $C_3$-$C_6$ ; un groupe halogéno-cycloalkyle en $C_3$-$C_6$ ; un groupe phényle ; ou un groupe phényle substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_{6}$-sulfinyle et un groupe halogéno-alkyl en $C_1$-$C_{6}$-sulfonyle)) ;

(2) lorsque $A^2$ est un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; un groupe alcynylène en $C_2$-$C_6$; un groupe halogéno-alcynylène en $C_3$-$C_6$ ; alors $R^7$ est un atome d'hydrogène ; un atome d'halogène ; un groupe halogéno-cycloalkyle en $C_3$-$C_6$ ; un groupe phényle ; un groupe phényle substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_6$-sulfinyle et un groupe halogéno-alkyl en $C_1$-$C_6$-sulfonyle ; ou un groupe de formule -$A^5$-$R^{12}$ (où $A^5$ est -O- ; -S- ; -SO- ou -$SO_2$-, ; et $R^{12}$ est un groupe cycloalkyle en $C_3$-$C_6$; un groupe halogéno-cycloalkyle en $C_3$-$C_6$ ; un groupe phényle ; un groupe phényle substitué

ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_6$-sulfinyle et un groupe halogéno-alkyl en $C_1$-$C_6$-sulfonyle ; ou un groupe de formule -$A^6$-$R^{14}$ (où $A^6$ est un groupe alkylène en $C_1$-$C_6$ ; un groupe halogéno-alkylène en $C_1$-$C_6$ ; un groupe alcénylène en $C_2$-$C_6$ ; un groupe halogéno-alcénylène en $C_2$-$C_6$ ; et $R^{14}$ est un atome d'hydrogène ; un atome d'halogène ; un groupe halogéno-cycloalkyle en $C_3$-$C_6$; un groupe halogéno-alcoxy en $C_1$-$C_6$ ; un groupe halogéno-alkylthio en $C_1$-$C_6$ ; un groupe halogéno-alkyl en $C_1$-$C_6$-sulfinyle ; un groupe halogéno-alkyl en $C_1$-$C_6$-sulfonyle ; un groupe phényle ; un groupe phényle substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_6$-sulfinyle et un groupe halogéno-alkyl en $C_1$-$C_6$-sulfonyle ; un groupe phénoxy ; un groupe phénoxy substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_6$-sulfinyle et un groupe halogéno-alkyl en $C_1$-$C_6$-sulfonyle ; un groupe phénylthio ; ou un groupe phénylthio substitué ayant au moins un substituant, qui est identique ou différent et est choisi dans le groupe constitué par un atome d'halogène, un groupe halogéno-alkyle en $C_1$-$C_6$, un groupe halogéno-alcoxy en $C_1$-$C_6$, un groupe halogéno-alkylthio en $C_1$-$C_6$, un groupe halogéno-alkyl en $C_1$-$C_6$-sulfinyle et un groupe halogéno-alkyl en $C_1$-$C_6$-sulfonyle)); et en outre,

$Z^1$ et $Z^2$ sont tels que définis dans la revendication 1 en ce qui concerne la formule (I-2).

**3.** Utilisation du dérivé diamide de l'acide phtalique selon la revendication 1 ou 2 en tant qu'insecticide agricole et horticole.

**4.** Procédé pour lutter contre des insectes nuisibles indésirables pour une culture utile, **caractérisé par** le traitement d'une culture en question avec une quantité efficace du dérivé diamide de l'acide phtalique selon la revendication 1 ou 2.

**5.** Insecticide agricole et horticole, comprenant le dérivé diamide de l'acide phtalique selon la revendication 1 ou 2 en tant qu'ingrédient actif.